# EUROPEAN PATENT APPLICATION

(11) **EP 4 578 407 A1**
(43) Date of publication of application: **02.07.2025**
(21) Application number: 23872156.7
(22) Date of filing: 22.09.2023
(51) Int. Cl.: A61B 17/04, A61B 17/12

(54) **LIGATION DEVICE**

(30) Priority: 30.09.2022 JP 2022159016; 30.09.2022 JP 2022159015
(71) Applicant: BROTHER KOGYO KABUSHIKI KAISHA, Nagoya-shi, Aichi 467-8561 (JP)
(72) Inventor: OSAWA, Naokatsu, Nagoya-shi, Aichi 467-8562 (JP); YAMANO, Junji, Nagoya-shi, Aichi 467-8562 (JP); IIJIMA, Ryuta, Nagoya-shi, Aichi 467-8562 (JP); ICHIHASHI, Masashi, Nagoya-shi, Aichi 467-8562 (JP)
(74) Representative: Prüfer & Partner mbB Patentanwälte · Rechtsanwälte
(86) International application number: PCT/JP2023/034443
(87) International publication number: WO 2024/070930

(57) **Abstract**

Provided is a ligation device that can readily wind a thread used for ligating a ligation object around a ligation object in ligation of the ligation object.

According to a ligation device 10, when a notch 44 of a ligation thread hook 34 or a notch 56 of a resetting thread hook 54 is located closer to a distal end of a second jaw portion 18 in a long side direction than an intersection position of a first thread-hook passage hole 38 and a thread feed rod passage hole 42 and a thread feed rod 64 is at a protruding position, a thread engagement portion 74 of the thread feed rod 64 is located further from a first jaw portion 12 than the first thread-hook passage hole 38 in a z-direction as a first short side direction and at a position overlapping the first thread-hook passage hole 38 in a y-direction, that is, a second short side direction. Thus, a thread T can be wound around an outer periphery of a ligation object 32 by holding the thread by a thread hook.

## Description

### TECHNICAL FIELD

The present invention relates to a ligation device that can ligate a ligation object with a thread, and more particularly to a ligation device that can readily wind a thread around an outer periphery of a ligation object prior to ligation of the ligation object.

### BACKGROUND

There has been proposed a suturing device capable of suturing a ligation object with a thread-like member wound around the ligation object instead of using metal staples in ligation of the ligation with a thread, for example, in a field of living body surgery. For example, the suturing devices proposed in Patent Literatures 1 and 2 are such a device.

In the suturing device proposed in Patent Literature 1, a U-shaped suture strip is attached for each securing operation, and a ligation object is ligated using the attached suture strip. According to the suturing device proposed in Patent Literature 1, the suture strip needs to be attached to the suturing device for each securing operation, thereby causing difficulty in ligation using a thread.

Patent Literature 2 describes a medical device that includes a proximal shaft, a flexible bending section, and a distal shaft that are connected to each other and through which a wire extends. A distal portion is connected to a position distal to the bending section or to a distal portion of the bending section. By pulling an operation wire at a proximal end, the bending section can be bent, allowing the wire to surround a ligation object such as a blood vessel. According to the medical device of Patent Literature 2, by pulling out the wire from a proximal end while the suture is connected to a distal end of the wire, the wire can be replaced with the suture that surrounds the ligation object, and by then transferring a knot tied outside a living body into the living body, the ligation object can be ligated.

### Citation List

### Patent Literature

[Patent Literature 1] JP2014-515637A
[Patent Literature 2] JP2017-158614A

### SUMMARY

### Technical Problem

However, neither Patent Literature 1 nor Patent Literature 2 discloses a technique of directly winding a thread around an outer periphery of a ligation object when ligating the ligation object with a thread.

The present invention has been made in view of the above circumstances, and an object of the present invention is to provide a ligation device capable of directly winding a thread for ligating a ligation object around an outer periphery of the ligation object when ligating the ligation.

### Solution to Problem

The gist of the present invention is that a ligation device includes (a) a columnar main body; a first jaw portion connected to one end of the main body in a longer side direction; a second jaw portion connected to the one end of the main body in the longer side direction such that the second jaw portion is movable between a holding position where the first jaw portion and the second jaw portion hold a ligation object therebetween and a separated position where the second jaw portion is separated from the first jaw portion; a thread feed rod including a thread engagement portion at a distal end of the thread feed rod, the thread engagement portion being configured to engage with a thread to be used for ligation of the ligation object, the thread feed rod being accommodated in the first jaw portion, the thread feed rod being movable between a retracted position where the thread engagement portion is located inside the first jaw portion and a protruding position where the thread engagement portion protrudes from the first jaw position when the second jaw portion is located at the holding position; and a thread pulling member disposed inside the main body, the thread pulling member including a thread hook portion at a distal end of the thread pulling member, the thread hook portion being configured to catch the thread engaged with the thread engagement portion, wherein (b) the second jaw portion has a hook passage hole extending along the long side direction, the hook passage hole through which the thread hook portion is insertable; and a thread feed rod passage hole through which the thread feed rod is insertable along a first short side direction, the first short side direction being a direction from the first jaw portion toward the second jaw portion and intersecting the long side direction, wherein (c) the hook passage hole and the thread feed rod passage hole partially intersect each other, and wherein (d) when the thread hook portion is located closer to a distal end of the second jaw portion in the long side direction than an intersecting position of the hook passage hole and the thread feed rod passage hole and the thread feed rod is located at the protruding position, the thread engagement portion of the thread feed rod is located downstream of the hook passage hole in the first short side direction, and is disposed at a position where the thread engagement portion overlaps the hook passage hole in a second short side direction intersecting the long side direction and the first short side direction.

### Advantageous Effects of Invention

According to the ligation device of the present invention, when the thread hook portion is located closer to the distal end of the second jaw portion in the long side direction than the intersection position of the thread hook passage hole and the thread feed rod passage hole and the thread feed rod is at the protruding position, the thread engagement portion of the thread feed rod is located further from the first jaw portion than the thread hook passage hole in the first short side direction and at the position overlapping the thread hook passage hole in the second short side direction that intersects the long side direction and the first short side direction. Thus, the thread can be directly wound around an outer periphery of the ligation object by pulling the thread pulling member while the thread hooked on the thread engagement portion of the thread feed rod is held by the thread hook portion located further toward the distal end of the second jaw portion in the long side direction than the intersection position of the hook passage hole and the thread feed rod passage hole.

### BRIEF DESCRIPTION OF THE DRAWINGS

[FIG. 1] FIG. 1 is a perspective view of a ligation device according to an embodiment of the present invention as viewed obliquely from above.
[FIG. 2] FIG. 2 is a plan view of the ligation device of FIG. 1.
[FIG. 3] FIG. 2 is a plan view of the ligation device of FIG. 1.
[FIG. 4] FIG. 4 is a rear view of the ligation device of FIG. 1 as viewed from its proximal end.
[FIG. 5] FIG. 5 is a vertical cross-sectional view of the ligation device of FIG. 1, that is, a cross-sectional view taken along line IV-IV of the ligation device of FIG. 2.
[FIG. 6] FIG. 5 is a horizontal cross-sectional view of the ligation device of FIG. 1, that is, a cross-sectional view taken along line V-V of the ligation device of FIG. 3.
[FIG. 7] FIG. 7 is a perspective view of a ligation thread hook used in the ligation device of FIG. 1.
[FIG. 8] FIG. 8 is a plan view illustrating a distal end portion of the ligation thread hook of FIG. 7.
[FIG. 9] FIG. 9 is an enlarged perspective view illustrating the distal end portion of the ligation thread hook of FIG. 7.
[FIG. 10] FIG. 10 is a plan view of a distal end portion of a resetting thread hook used in the ligation device of FIG. 1.
[FIG. 11] FIG. 11 is a cross-sectional view illustrating a cross section of the resetting thread hook of FIG. 10.
[FIG. 12] FIG. 12 is a perspective view of a thread feed rod used in the ligation device of FIG. 1.
[FIG. 13] FIG. 13 illustrates a positional relationship between the thread feed rod of FIG. 12 and the distal end portion of the ligation thread hook of FIG. 8.
[FIG. 14] FIG. 14 illustrates a positional relationship between the thread feed rod of FIG. 12 and the distal end portion of the resetting thread hook of FIG. 10.
[FIG. 15] FIG. 15 is a perspective view illustrating a pair of thread loop shafts in the ligation device of FIG. 1.
[FIG. 16] FIG. 16 illustrates a ligation object holding step in which a ligated object is held between a lower jaw portion and an upper jaw portion during operation of the ligation device of FIG. 1.
[FIG. 17] FIG. 17 illustrates a knot pusher forward moving step in which a knot pusher and the ligation thread hook are moved forward toward the ligation object in a thread holding operation of the ligation device of FIG. 1.
[FIG. 18] FIG. 18 illustrates a ligation thread hook protruding step in which the ligation thread hook that holds a distal thread relative to the ligation object is caused to protrude from the knot pusher in the thread holding operation of the ligation device of FIG. 1.
[FIG. 19] FIG. 19 illustrates a thread feed rod protruding step in which the thread feed rod is upwardly moved until the thread feed rod protrudes the upper jaw portion in the thread holding operation of the ligation device of FIG. 1.
[FIG. 20] FIG. 20 illustrates a thread holding step in which the thread hook holding member is caused to protrude from a tubular body of the ligation thread hook to catch and loosely hold the distal thread relative to the ligation object and the thread hook holding member is moved backward slightly in the thread holding operation of the ligation device of FIG. 1.
[FIG. 21] FIG. 21 illustrates a knot pusher backward moving step in which the thread feed rod is moved downward and the knot pusher and the ligation thread hook are moved backward in the thread holding operation of the ligation device of FIG. 1.
[FIG. 22] FIG. 22 illustrates a first thread cutting step in which the thread is securely held by the thread hook holding member, and then the thread feed rod is moved upward until the thread feed rod protrudes from the upper jaw portion to cut the thread in a thread cutting operation of the ligation device of FIG. 1.
[FIG. 23] FIG. 23 illustrates a thread drawing step in which the thread is loosely held by the thread hook holding member and then the ligation thread hook and the knot pusher are moved backward to a proximal end portion of the ligation device to cause the thread to extend through two thread loops in the thread cutting operation of the ligation device of FIG. 1.
[FIG. 24] FIG. 24 illustrates a thread loop removing step in which the thread loops wound around the respective thread loop shafts are removed therefrom by a reverse rotation of the pair of thread loop shafts while tension is applied to the thread by a forward movement of the ligation thread hook and a backward movement of the resetting thread hook, in a ligation operation of the ligation device of FIG. 1.
[FIG. 25] FIG. 25 illustrates a thread loop moving step in which the two thread loops are moved forward to tighten the thread loops y simultaneously moving the knot pusher and the ligation thread hook forward toward the ligation object, and the resetting thread hook backward, in the ligation operation of the ligation device of FIG. 1.
[FIG. 26] FIG. 26 illustrates a knot forming step in which a knot is formed by moving the resetting thread hook forward and the ligation thread hook backward to move the loop closer to the knot pusher in the ligation operation of the ligation device of FIG. 1.
[FIG. 27] FIG. 27 is an enlarged view of the thread loop before the knot forming step of FIG. 26 is performed.
[FIG. 28] FIG. 28 is an enlarged view illustrating a state where one of the two thread loops, which is closer to the knot pusher, is converted after the knot forming step of FIG. 26.
[FIG. 29] FIG. 29 illustrates a second thread cutting step in which two threads extending between the knot and the ligation thread hook and between the knot and the resetting thread hook are cut simultaneously by rotating a second cutter to a cutting position in a state where the knot pusher is retracted in the ligation operation of the ligation device of FIG. 1.
[FIG. 30] FIG. 30 illustrates a thread collecting step in which the thread is collected by moving the second cutter backward from the cutting position and moving the knot pusher and the ligation thread hook backward to a rear end in the ligation operation of the ligation device of FIG. 1.
[FIG. 31] FIG. 31 illustrates a ligation object removing step in which the ligation object is removed by opening and closing the upper jaw portion in the ligation operation of the ligation device of FIG. 1.
[FIG. 32] FIG. 32 illustrates a resetting thread hook forward moving step in which the resetting thread hook is moved forward to the distal end portion of the ligation device in a resetting operation of the ligation device of FIG. 1.
[FIG. 33] FIG. 33 illustrates a thread feed rod protruding step in which the thread feed rod is upwardly moved until the thread feed rod protrudes the upper jaw portion in the ligation operation of the ligation device of FIG. 1 in the resetting operation of the ligation device of FIG. 1.
[FIG. 34] FIG. 34 illustrates a thread holding step in which the thread hook holding member is caused to protrude from the tubular body of the resetting thread hook to catch and securely hold the thread raised by the thread feed rod and the resetting thread hook is moved backward slightly in the resetting operation of the ligation device of FIG. 1.
[FIG. 35] FIG. 35 illustrates a rod-guide-groove thread setting step in which the thread feed rod is lowered to be accommodated in the lower jaw portion, the resetting thread hook is moved backward such that the resetting thread hook is located closer to the proximal end of the ligation device than the pair of thread loop shafts, and the thread is set in the rod guide grooves for guiding the resetting thread hook of the pair of thread loop shafts in the resetting operation of the ligation device of FIG. 1.
[FIG. 36] FIG. 36 illustrates a thread groove thread setting step in which the thread extending in the rod guide groove is set in the thread groove extending in a direction orthogonal to the rod guide groove by rotating the pair of thread loop shafts by 90 degrees in a first rotation direction in the resetting operation of the first ligation device.
[FIG. 37] FIG. 37 illustrates a thread loop forming step in which two thread loops are formed by rotating the pair of thread loop shafts by 270 degrees in a second rotation direction opposite to the first rotation direction to wind the thread around each of the thread loop shafts in the resetting operation of the first ligation device.
[FIG. 38] FIG. 38 illustrates a thread hook placing step in which the knot pusher is moved forward while being rotated about the axis and passing through the thread of the two thread loops wound around the thread loop shafts, respectively, in the resetting operation of the first ligation device.
[FIG. 39] FIG. 39 illustrates the thread hook placing step of FIG. 38 in which the knot pusher is approaching a circumferential thread of the thread loop located toward the proximal end.
[FIG. 40] FIG. 40 illustrates the thread hook placing step of FIG. 38 in which while the knot pusher is moving forward, the knot pusher is rotated about the axis so that an inclined surface formed at a distal end portion of the knot pusher is caused to pass above the circumferential thread of the thread loop located toward the proximal end.
[FIG. 41] FIG. 41 illustrates the thread hook placing step of FIG. 38 in which while the knot pusher is moving forward, the knot pusher is rotated about the axis so that the inclined surface formed at the distal end portion of the knot pusher is caused to pass through a radial thread of the thread loop located toward the proximal end.
[FIG. 42] FIG. 42 illustrates the thread hook placing step of FIG. 38 in which while the knot pusher is moving forward, the knot pusher is rotated about the axis so that the inclined surface formed at the distal end portion of the knot pusher is caused to pass above the circumferential thread of the thread loop located toward the proximal end and a circumferential thread of the thread loop located toward the distal end.
[FIG. 43] FIG. 43 illustrates the thread hook placing step of FIG. 38 in which while the knot pusher is moving forward, the knot pusher is rotated about the axis so that the inclined surface formed at the distal end portion of the knot pusher is caused to pass through the radial thread of the thread loop located toward the distal end.
[FIG. 44] FIG. 44 illustrates the thread hook placing step of FIG. 38 in which while the knot pusher is moving forward, the knot pusher is rotated about the axis so that the inclined surface formed at the distal end portion of the knot pusher is caused to pass above the circumferential thread of the thread loop located toward the distal end.

### DETAILED DESCRIPTION

Hereinafter, an embodiment of the present invention will be described in detail with reference to the drawings.

### Embodiments

### (Configuration of Ligation Device 10)

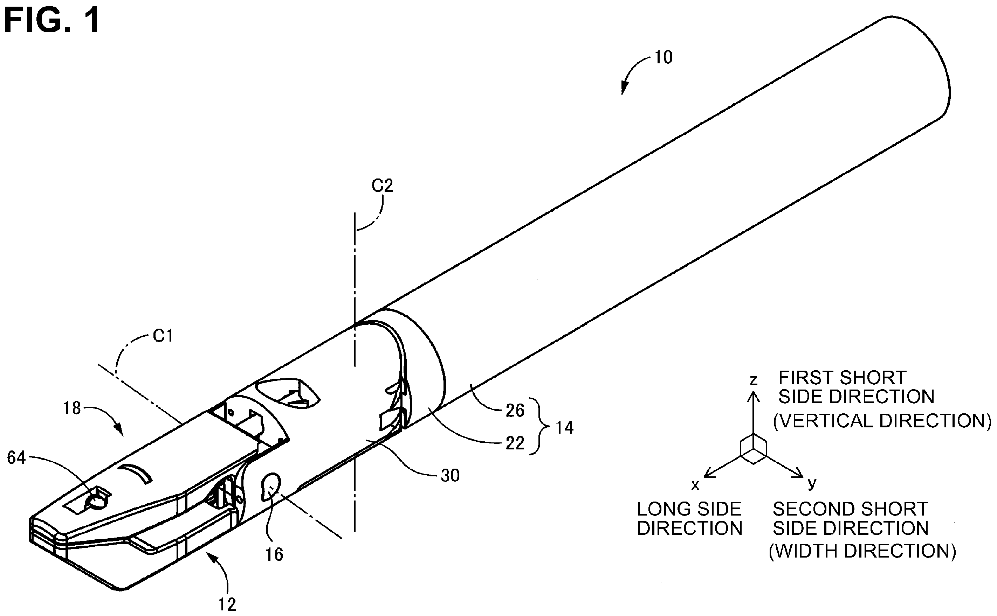

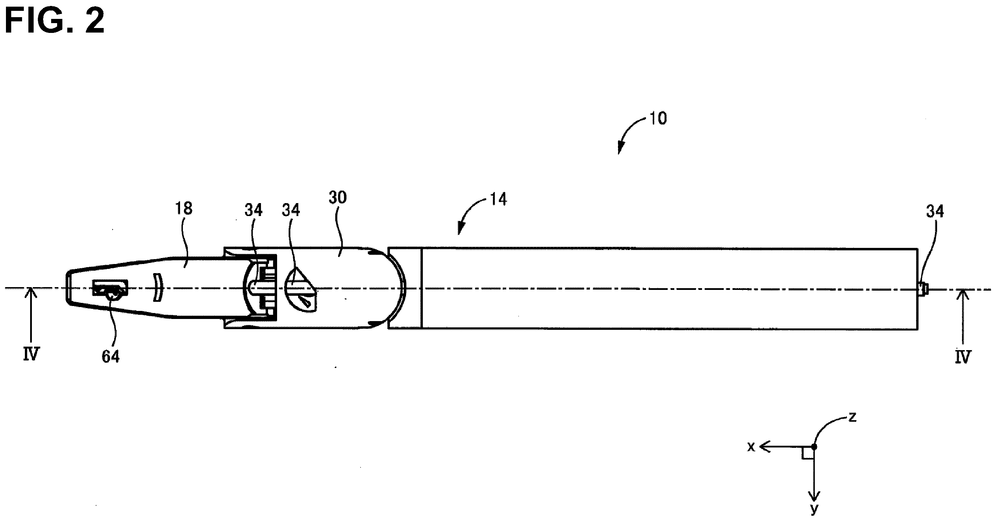

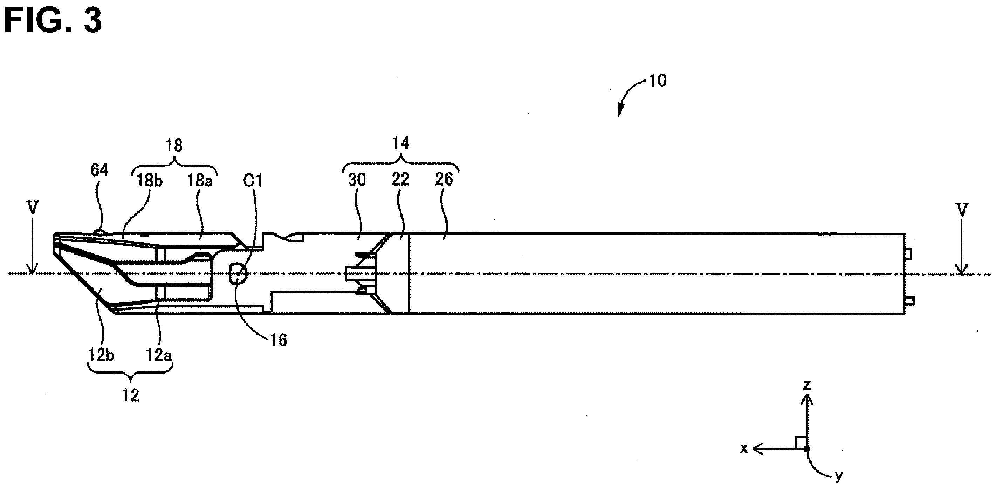

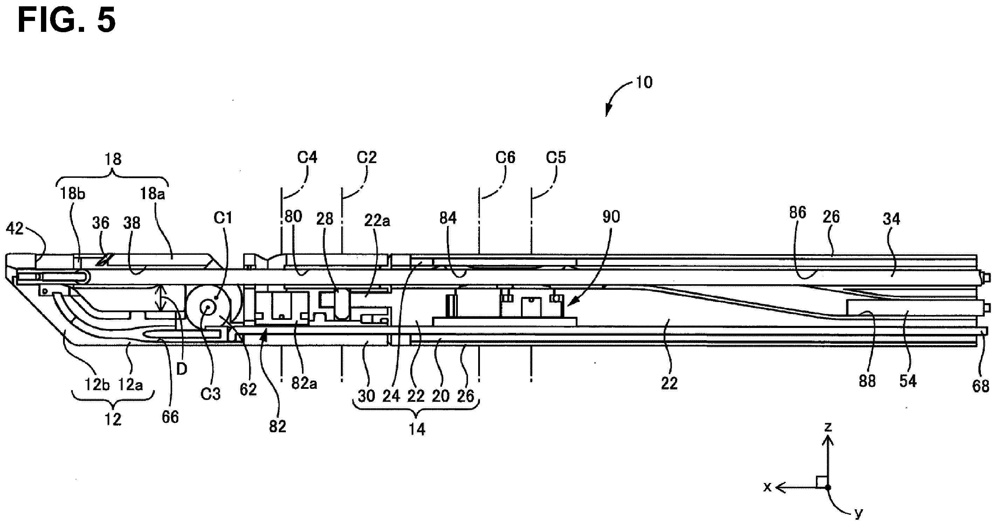

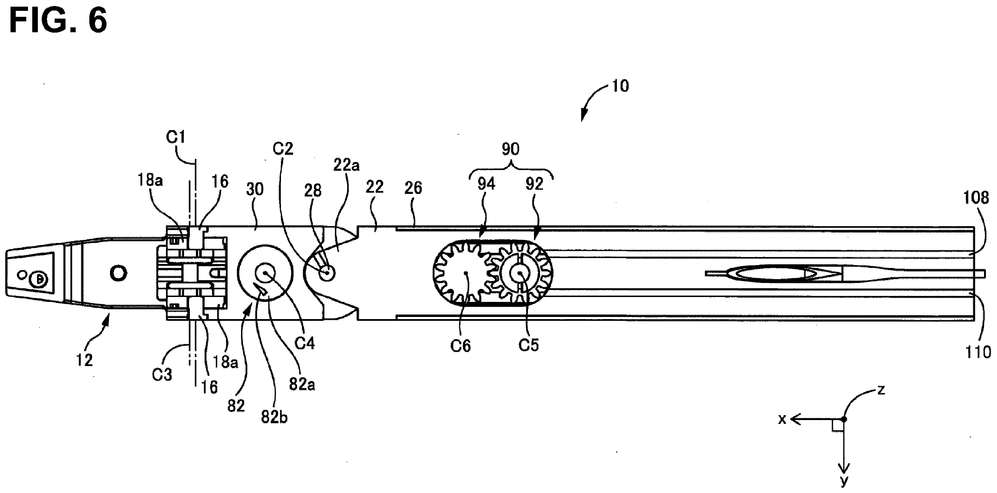

FIGS. 1, 2, 3, and 4 illustrate a ligation device 10 according to one embodiment of the present invention, wherein FIGS. 1, 2, 3, and 4 are a perspective view, a plan view, a side view, and a rear view, that is, a rear end view, of the ligation device 10 in which a second jaw portion 18 is closed. FIG. 5 is a cross-sectional view taken along line IV-IV of FIG. 2. FIG. 6 is a cross-sectional view taken along line V-V of FIG. 3.

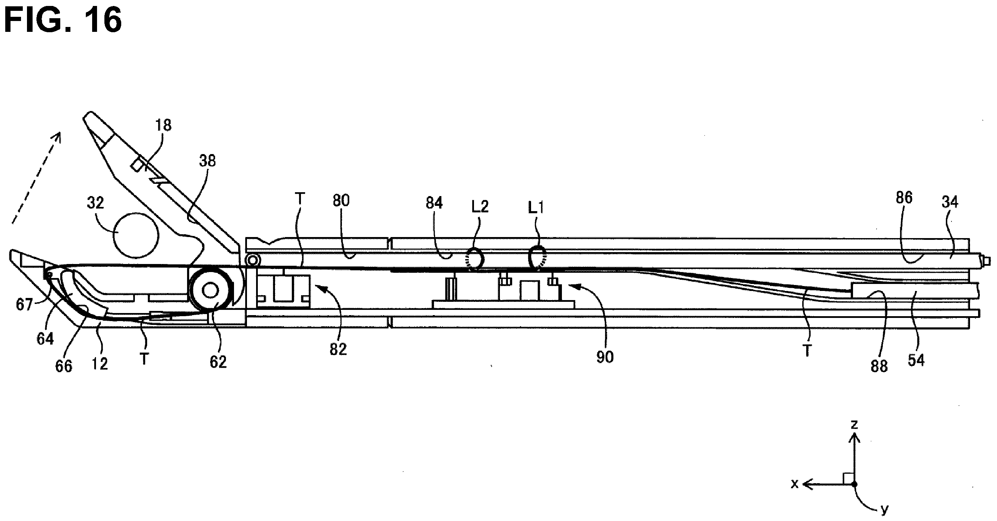

The ligation device 10 has a longitudinal shape as a whole, and a distal end portion thereof opens and closes between a separated position and a holding position. As a result, as illustrated in FIG. 16 and subsequent drawings, a knot M can be formed in a thread T wound around a ligation object 32 at the holding position where a part of a living body, for example, the ligation object 32 such as a blood vessel is held. The ligation device 10 also serves as a tightening device for fastening the ligation object 32 using the thread T. In the present embodiment, as illustrated in FIG. 1, a long side direction of the ligation device 10 is defined as an x-direction, a first short side direction perpendicular to the x-direction and parallel to an opening surface of a distal end portion of the ligation device 10, that is, a vertical direction is defined as a z-direction, and a second short side direction perpendicular to the x-direction and the z-direction, that is, a width direction is defined as a y-direction.

The ligation device 10 includes a columnar main body 14, a holding portion, and a thread pulling member. The holding portion is connected to one end of the main body 14 in the x-direction and holds the ligation object 32. The holding portion includes a first jaw portion 12 and a second jaw portion 18. The first jaw portion 12 is connected to the one end of the main body 14 in the x-direction. The second jaw portion 18 moves between the holding position where the second jaw portion 18 and the first jaw portion 12 hold the ligation object 32 therebetween and the separated position where the second jaw portion 18 is separated from the first jaw portion 12. In the present embodiment, the first jaw portion 12 is disposed below the second jaw portion 18 in the z-direction, that is, in the vertical direction. Details of the first jaw portion 12 and the second jaw portion 18 will be described later. Hereinafter, the first jaw portion 12 may be also referred to as a lower jaw portion 12, and the second jaw portion 18 may be also referred to as an upper jaw portion 18. The thread pulling member is disposed to be movable along the x-direction in the main body 14, and pulls the thread for ligating the ligation object 32 into the main body 14. The thread pulling member is a first thread pulling member or a second thread pulling member described later. The ligation device 10 further includes a thread feed rod 64. The thread feed rod 64 is accommodated in the first jaw portion 12. The thread feed rod 64 has, at its distal end, a thread engagement portion 74 to be engaged with the thread T for ligating the ligation object 32. The thread feed rod 64 moves between a retracted position where the thread engagement portion 74 is located inside the first jaw portion 12 and a protruding position where the thread engagement portion 74 protrudes from the first jaw portion 12 when the second jaw portion 18 is located at the holding position. The thread feed rod 64 will be described in detail later. The ligation device 10 further includes a thread loop forming portion 90. The thread loop forming portion 90 forms two thread loops in the thread T that has been pulled into the main body 14 by the thread pulling member. The thread loop forming portion will be described in detail later.

The ligation device 10 includes an oscillation portion 30. The oscillation portion 30 is a coupling member that couples the first jaw portion 12 and the second jaw portion 18 to the main body 14. More specifically, the first jaw portion 12 is integral with a lower side of a distal end portion of the oscillation portion 30. The second jaw portion 18 has a pair of proximal end portions 18a spaced apart from each other in the y-direction. The pair of proximal end portions 18a are connected to the distal end portion of the oscillation portion 30 using a pair of first pins 16 so as to be rotatable about a first rotation axis C1 parallel to the y-direction. The second jaw portion 18 moves between the holding position where the second jaw portion 18 and the first jaw portion 12 hold the ligation object therebetween and the separated position where the second jaw portion 18 is separated from the first jaw portion 12. The main body 14 includes a lower main body portion 20, an intermediate main body portion 22, and an upper main body portion 24 that are disposed one above another in the z-direction, and a cylindrical retaining cylinder 26 that retains the lower main body portion 20, the intermediate main body portion 22, and the upper main body portion 24 fitted therein. A proximal end of the oscillation portion 30 is connected to a connecting plate portion 22a protruding from a distal end portion of the intermediate main body portion 22 via a second pin 28 so as to be rotatable about a second rotation axis C2 parallel to the z-direction. This configuration thus enables the first jaw portion 12 and the second jaw portion 18 to be opened and closed. The first jaw portion 12 and the second jaw portion 18 are connected to the main body 14 so as to oscillate with respect to the main body 14, thereby increasing flexibility in the operation of the first jaw portion 12 and the second jaw portion 18.

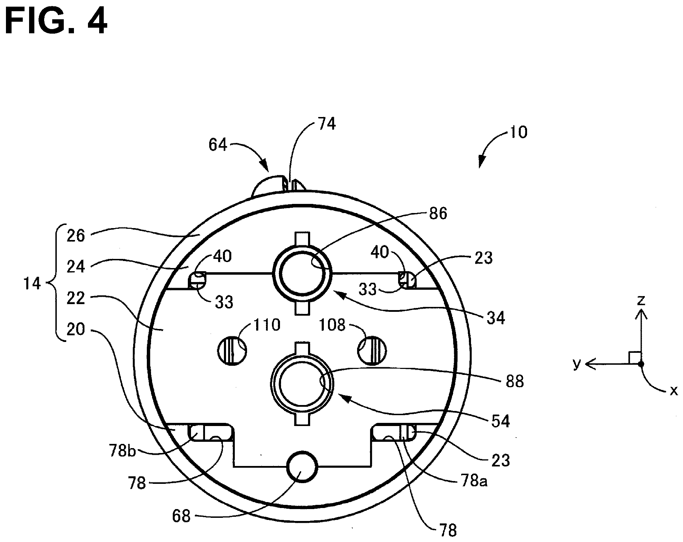

The oscillation portion 30 is operated to oscillate about the second rotation axis C2 via a pair of oscillation operation wires 33 whose one ends are fixed to the oscillation portion 30. Each of the oscillation operation wires 33 extends through a corresponding one of a pair of oscillation operation wire passage holes 40 opened between the intermediate main body portion 22 and the upper main body portion 24 on a proximal end of the ligation device 10 illustrated in FIG. 4. The second jaw portion 18 is operated to be opened and closed about the first rotation axis C1 via a pair of opening/closing operation wires 23 whose one ends are fixed to the second jaw portion 18. One of the opening/closing operation wires 23 extends through one of the pair of oscillation operation wire passage holes 40 and the other of the opening/closing operation wires 23 extends through the cutter operation wire 78a of the pair of cutter operation wires 78a, and 78b on the proximal end of the ligation device 10 illustrated in FIG. 4.

The first jaw portion 12 integrally protrudes from the oscillation portion 30 toward the distal end of the ligation device 10. A proximal end portion 12a of the first jaw portion 12 is formed to extend in parallel to the proximal end portion 18a of the second jaw portion 18 in a state where a distal end of the first jaw portion 12 and a distal end of the second jaw portion 18 are in contact with each other. A distal end portion 12b of the first jaw portion 12 obliquely extends toward the distal end portion 18b of the second jaw portion 18 as the distal end portion 12b extends toward its distal end in the long side direction x. The first jaw portion 12 is curved as a whole. A predetermined gap D for placing a ligation object 32 therein is provided between the proximal end portion 12a of the first jaw portion 12 and the proximal end portion 18a of the second jaw portion 18.

As illustrated in FIGS. 5 and 6, the second jaw portion 18 has a linear first thread-hook passage hole 38 therein that extends along the long side direction of the ligation device 10, that is, the x-direction. The first thread-hook passage hole 38 is provided to selectively guide a ligation thread hook 34 functioning as a first thread pulling member and a resetting thread hook 54 functioning as a second thread pulling member to the distal end of the ligation device 10. The second jaw portion 18 has a thread feed rod passage hole 42 extending through the second jaw portion 18. The thread feed rod passage hole 42 is configured such that the thread feed rod 64 is inserted therethrough along a direction from the first jaw portion 12 toward the second jaw portion 18, that is, a first short side direction orthogonal to the x-direction that is the long side direction of the ligation device 10, that is, the z-direction. The first thread-hook passage hole 38 and the thread feed rod passage hole 42 partially cross each other in their cross-sectional areas.

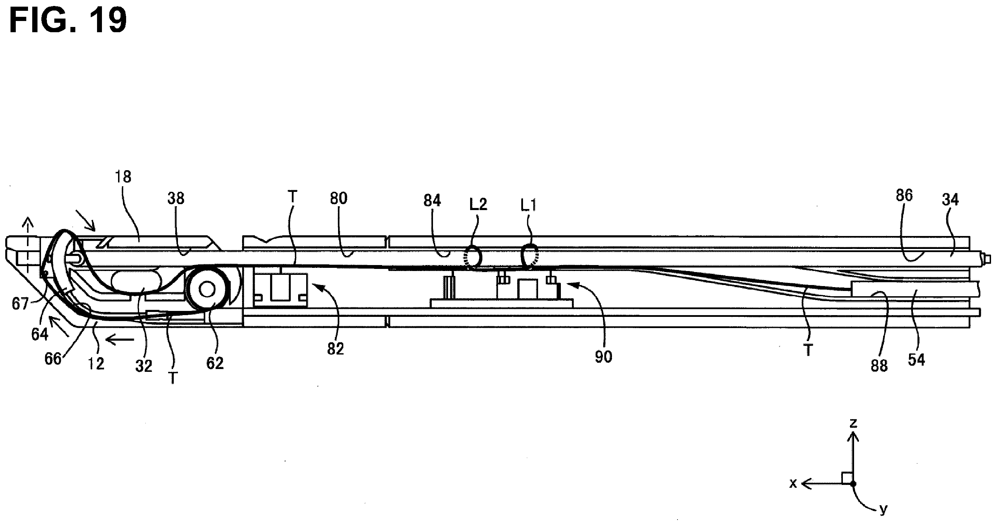

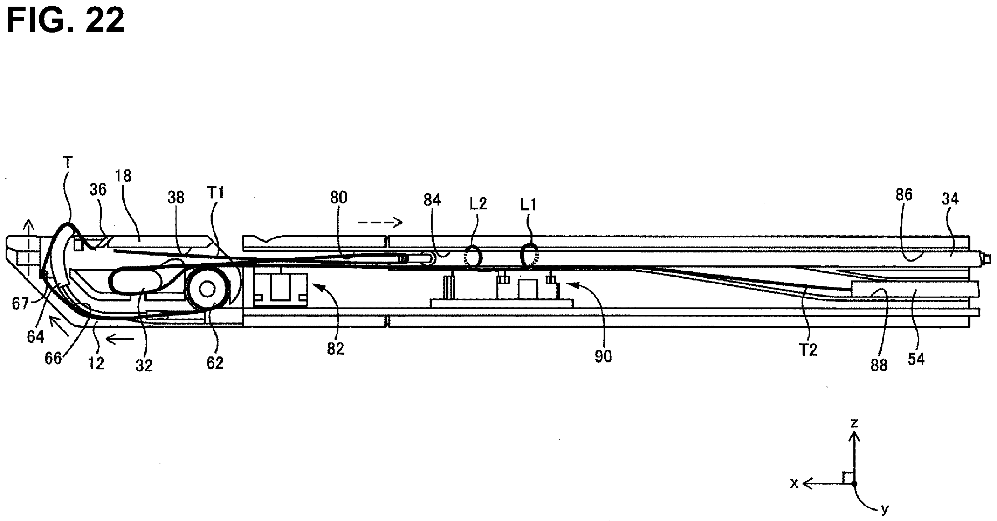

A first cutter 36 is fixed to the second jaw portion 18 between the thread feed rod passage hole 42 and the main body 14. The first cutter 36 cuts a thread T when the thread T hooked on a thread engagement portion 74 of the thread feed rod 64 is lifted in the z-direction by the thread feed rod 64. Thus, when the second jaw portion 18 is at the holding position, in response to the thread feed rod 64 reaching a second protruding position illustrated in FIG. 22 from a first protruding position illustrated in FIG. 19 where the thread feed rod 64 slightly protrudes from the thread feed rod passage hole 42 by further movement in the z-direction in order to cause the ligation thread hook 34 to hold the thread T, the thread T hooked on the thread engagement portion 74 of the thread feed rod 64 is lifted in the z-direction, whereby the thread T is cut by the first cutter 36 without moving the first cutter 36.

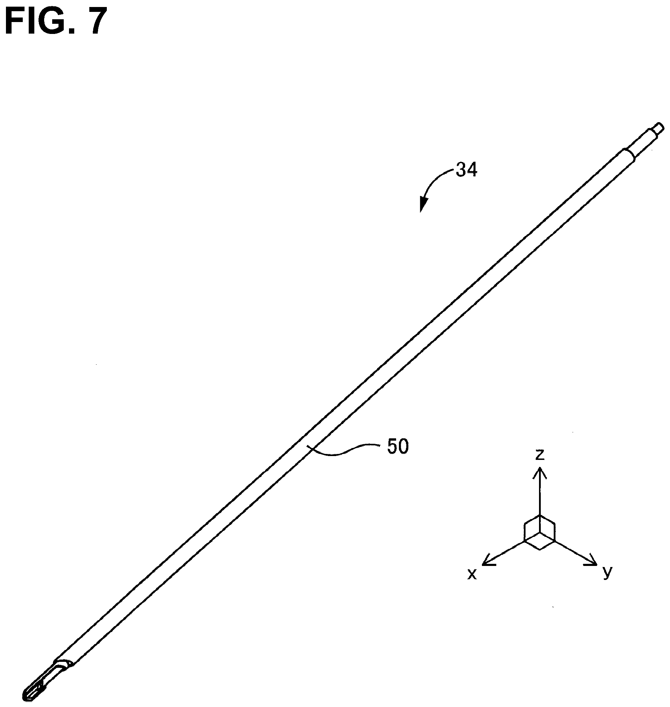

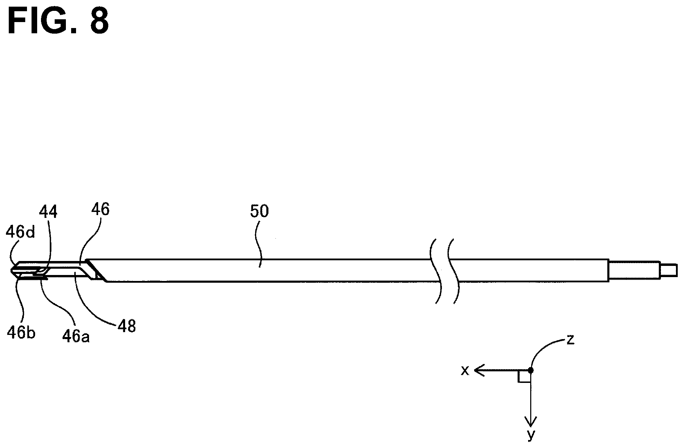

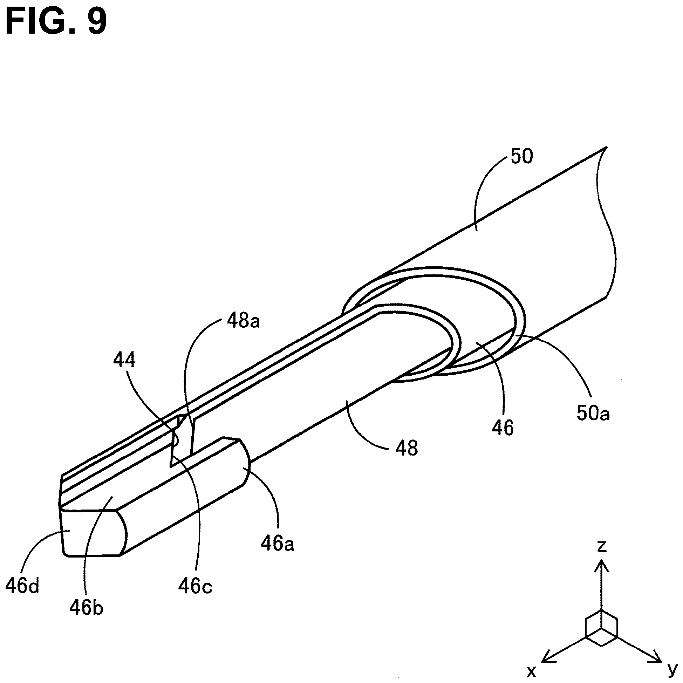

FIG. 7 is a perspective view of the ligation thread hook 34. FIG. 8 is a plan view of a distal end portion of the ligation thread hook 34. FIG. 9 is an enlarged perspective view of the distal end portion of the ligation thread hook 34. The ligation thread hook 34 includes a tubular body 46 having a notch 44 constituting a thread hook portion for hooking the thread T at its tip, and a columnar thread hook holding member 48 that is slidably inserted into the tubular body 46 and is configured to hold the thread T between the notch 44 and itself. The ligation thread hook 34 is made of a deformable material such as metal or plastic as a whole. The thread T hooked on the notch 44 is held by the thread hook holding member 48.

The tubular body 46 has an inclined surface 46c that forms an acute angle with respect to the x-direction and the y-direction. The tubular body 46 has a hooking projection 46a linearly projecting in a direction away from a distal end of the tubular body 46 from an end of the inclined surface 46c in the x-direction. The notch 44 is between the inclined surface 46c and the hooking projection 46a. In other words, the notch 44 is in the tubular body 46 such that a depth of the notch 44 in the y-direction decreases toward the distal end of the tubular body 46 in the x-direction when viewed from the radially outer side. The thread hook holding member 48 has an inclined surface 48a at its distal end. The inclined surface 48a is inclined with respect to the x-direction and the y-direction and forms an acute angle. The inclined surface 48a faces the inclined surface 46c of the tubular body 46, and is parallel to the inclined surface 46c. Thus, the thread T is reliably held.

The tubular body 46 has a flat portion 46b at its distal end portion. The flat portion 46b has a thickness less than an inner diameter of the tubular body 46. The flat portion 46b has the inclined surface 46c parallel to the inclined surface 48a at a portion thereof facing the inclined surface 48a of the thread hook holding member 48 such that the inclined surface 46c can surface contact with the inclined surface 48a of the thread hook holding member 48. As a result, the inclined surface 46c parallel to the inclined surface 48a comes into surface contact with the inclined surface 48a of the thread hook holding member 48. This prevents the thread hook holding member 48 from rotating about an axis with respect to the tubular body 46, thereby reliably holding the thread T between the inclined surface 48a of the thread hook holding member 48 and the inclined surface 46c of the flat portion 46b. The flat portion 46b has a thickness less than the inside diameter of the tubular body 46. Thus, the thread T held between the inclined surface 48a of the thread hook holding member 48 and the inclined surface 46c of the flat portion 46b can be pulled into the tubular body 46 and a knot pusher 50.

The ligation thread hook 34 includes the tubular knot pusher 50 into which the tubular body 46 is slidably inserted and into which the tubular body 46 is slidably and projectably inserted. The knot pusher 50 is mounted concentrically with the tubular body 46, and functions as an outer tubular member of the ligation thread hook 34. The knot pusher 50 has an inclined end face 50a at is distal end. The inclined end face 50a has an opening that allows the tubular body 46 to protrude therefrom.

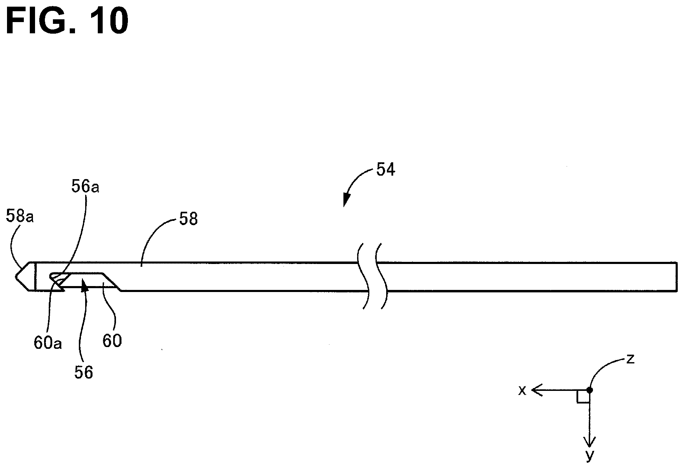

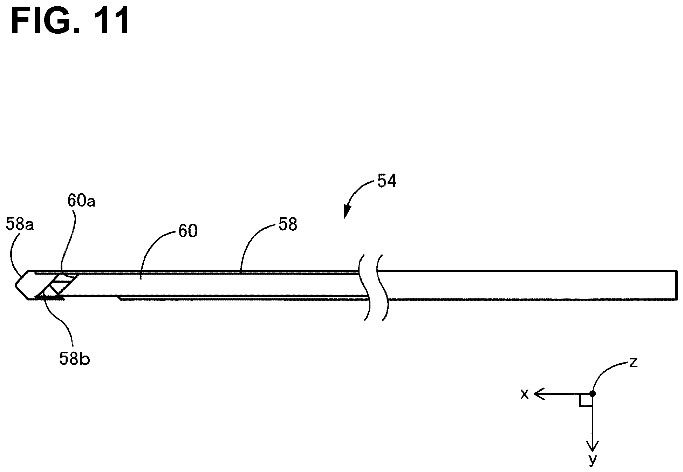

FIG. 10 is a plan view of a distal end portion of the resetting thread hook 54 corresponding to the second thread pulling member. FIG. 11 is a cross-sectional view of the distal end portion of the resetting thread hook 54. The resetting thread hook 54 includes a tubular body 58 having a notch 56 constituting a thread hook portion for hooking the thread T, and a columnar thread hook holding member 60 that is slidably inserted into the tubular body 58 and is configured to hold the thread T between the notch 56 and itself. The resetting thread hook 54 is made of a deformable material such as metal or plastic as a whole. A moving path of the ligation thread hook 34 and a moving path of the resetting thread hook 54 are coaxial in the first thread-hook passage hole 38, a second thread-hook passage hole 80, and a third thread-hook passage hole 84. The moving path of the ligation thread hook 34 and the moving path of the resetting thread hook 54 are coaxial at positions intersecting a fifth rotation axis C5 and a sixth rotation axis C6 that are rotational centers of a first thread loop shaft 92 and a second thread loop shaft 94, respectively, interposed in series in the third thread-hook passage hole 84.

A distal end portion 56a of the notch 56 formed in the tubular body 58 is formed such that a cut depth increases in the y-direction as viewed from the radially outer side toward the distal end of the tubular body 46 in the x-direction. The thread hook holding member 60 has an inclined surface 60a at its distal end portion. The inclined surface 60a is inclined toward the distal end as the notch 56 is shallower, and forms an acute angle with respect to the x-direction. The inclined surface 60a is reversely inclined with respect to the distal end portion 56a of the notch 56.

The tubular body 58 has the inclined surface 58b parallel to the inclined surface 60a at the distal end portion that is closer to the distal end than the notch 56 in the tubular body 58 and at a portion thereof facing the inclined surface 60a of the thread hook holding member 60. The inclined surface 58b is formed such that the inclined surface 58b can surface contact with the inclined surface 60a of the thread hook holding member 60.

A bobbin 62 around which the thread T is wound is supported in the proximal end portion 12a of the first jaw portion 12 so as to be rotatable about a third rotation axis C3 parallel to the y-direction that is the second short side direction. The bobbin 62 functions as a thread storage portion. The bobbin 62 is disposed between two rotating shafts 16 in the y-direction, and between the thread engagement portion 74 of the thread feed rod 64 and the main body 14 in the x-direction that is the long side direction of the ligation device 10. A thread feed rod accommodating hole 66 for slidably accommodating the thread feed rod 64 is defined in the first jaw portion 12 along the long side direction of the first jaw portion 12. Further, a hook pin 67 for holding the thread T is disposed at the distal end portion of the first jaw portion 12 in an opening of the thread feed rod accommodating hole 66.

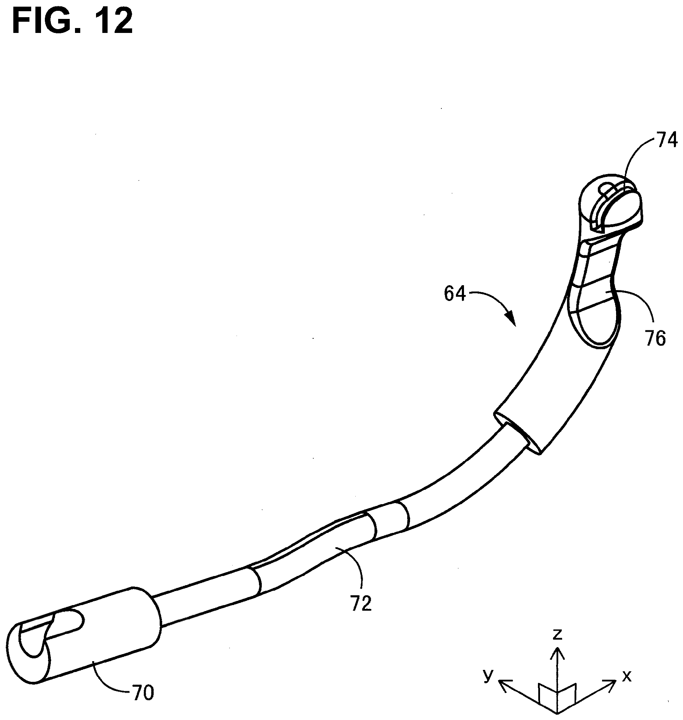

FIG. 12 is a perspective view of the thread feed rod 64, a cylindrical connecting member 70 connected to the thread feed rod operation wire 68 of FIG. 5, and a flexible coupling member 72 coupling the thread feed rod 64 and the connecting member 70. The coupling member 72 is made of a flexible member such as a synthetic resin or a coil spring. The thread feed rod 64 and the connecting member 70 are offset such that the center of the thread feed rod 64 is shifted in the y-direction from the center of the first thread-hook passage hole 38 in the thread feed rod accommodating hole 66. The thread feed rod operation wire 68 is operated using an end portion exposed from the opening of the proximal end of the ligation device 10 illustrated in FIG. 4. In response to the end portion of the thread feed rod operation wire 68 being operated to be retracted, the thread feed rod 64 is located at a retracted position where the thread feed rod 64 is accommodated in the thread feed rod accommodating hole 66. In response to the end portion of the thread feed rod operation wire 68 being operated to protrude, the thread feed rod 64 is located at a protruding position where the thread engagement portion 74 of the thread feed rod 64 protrudes from the thread feed rod accommodating hole 66.

The thread feed rod 64 is curved such that the thread feed rod 64 curvedly extends toward its distal end in the z-direction that is the first short side direction. The thread feed rod 64 has the groove-shaped thread engagement portion 74 into which the thread T is engaged is formed along the x-direction that is the long side direction of the ligation device 10. The thread feed rod 64 has a recessed groove 76 at a side surface of the distal end portion thereof. The recessed groove 76 extends through the side surface in the x-direction. The recessed groove 76 is for avoiding interference between the ligation thread hook 34 and the resetting thread hook 54 that are located in the linear first thread-hook passage hole 38 extending along the x-direction in the second jaw portion 18 when the distal end of the thread feed rod 64 protrudes from the thread feed rod accommodating hole 66 and is located in the thread feed rod passage hole 42 of the second jaw portion 18. This enables the thread T to be hooked on the thread engagement portion 74 readily.

In the thread feed rod 64, the recessed groove 76 is defined upstream of the thread engagement portion 74 in the z-direction, that is, the first short side direction, that is, closer to the first jaw portion 12 than the thread engagement portion 74, and at a position overlapping the thread engagement portion 74. The recessed groove 76 is formed in the thread feed rod 64 at a position that overlaps with the first thread-hook passage hole 38 in the z-direction that is the first short side direction when the thread feed rod 64 is at the protruding position. As a result, the thread engagement portion 74 of the thread feed rod 64 can cross the notch 44 of the ligation thread hook 34 or the notch 56 of the resetting thread hook 54 in the y-direction that is the second short side direction.

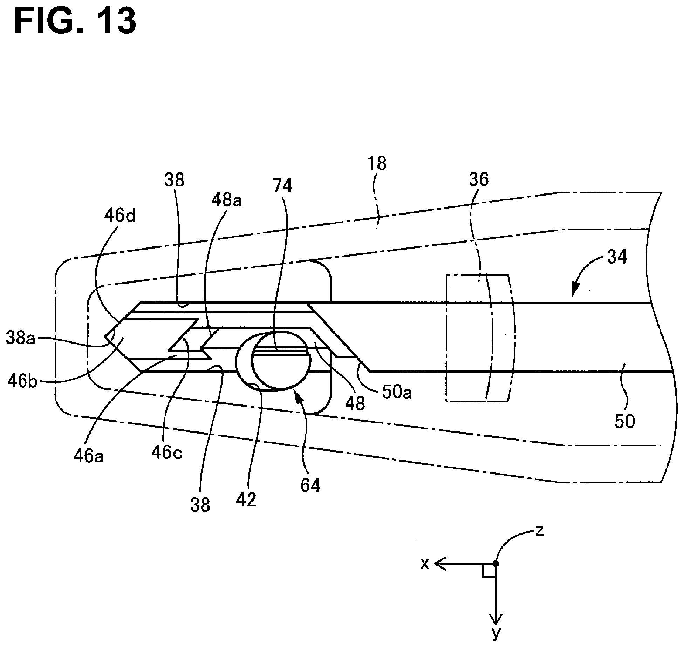

FIG. 13 illustrates a relationship between the ligation thread hook 34 and the thread feed rod 64 when the ligation thread hook 34 has reached its farthest position on the distal end side of the ligation device 10, a distal end portion of the notch 44 functioning as the thread hook portion of the ligation thread hook 34 is positioned further to the distal end side of the second jaw portion 18 in the long side direction than the intersecting position of the first thread-hook passage hole 38 and the thread feed rod passage hole 42, and the thread feed rod 64 is in its most protruding position where the thread feed rod 64 protrudes the furthest from the thread feed rod passage hole 42 in the z-direction. When the thread feed rod 64 is in the protruding position, the thread engagement portion 74 formed at the distal end portion of the thread feed rod 64 is positioned above, that is, downstream of the first thread-hook passage hole 38 in the z-direction that is the first short side direction and positioned to overlap the first thread-hook passage hole 38 in the y-direction that is the second short side direction. The recessed groove 76 of the thread feed rod 64 is positioned to overlap the first thread-hook passage hole 38 in both the z-direction and the y-direction. That is, the thread feed rod 64 has the recessed groove 76 at a position below the thread engagement portion 74 in the z-direction and at a position overlapping the thread engagement portion 74 in the y-direction. The recessed groove 76 overlaps the first thread-hook passage hole 38 in the z-direction when the thread feed rod 64 is in the protruding position. Thus, the thread engagement portion 74 of the thread feed rod 64 and the notch 44 constituting the thread hook portion of the ligation thread hook 34 can cross each other, and a thread T engaged with the thread engagement portion 74 formed at the distal end of the thread feed rod 64 is hooked on the notch 44 functioning as the thread hook portion of the ligation thread hook 34.

In FIG. 13, the first thread-hook passage hole 38 has a V-shaped distal end bottom 38a including a pair of inclined surfaces, where a central portion of the distal end bottom 38a is closer to the distal end in plan view on an x-y plane. Similarly, a distal end of the flat portion 46b has a V-shaped distal end surface 46d including a pair of inclined surfaces, where a central portion of the distal end surface 46d is closer to the distal end in plan view on the x-y plane. Thus, the ligation thread hook 34 is moved forward to the distal end to contact the distal end surface 46d formed at the distal end of the flat portion 46 with the distal end bottom 38a of the first thread-hook passage hole 38, whereby a rotational phase around an axis of the tubular body 46 is aligned.

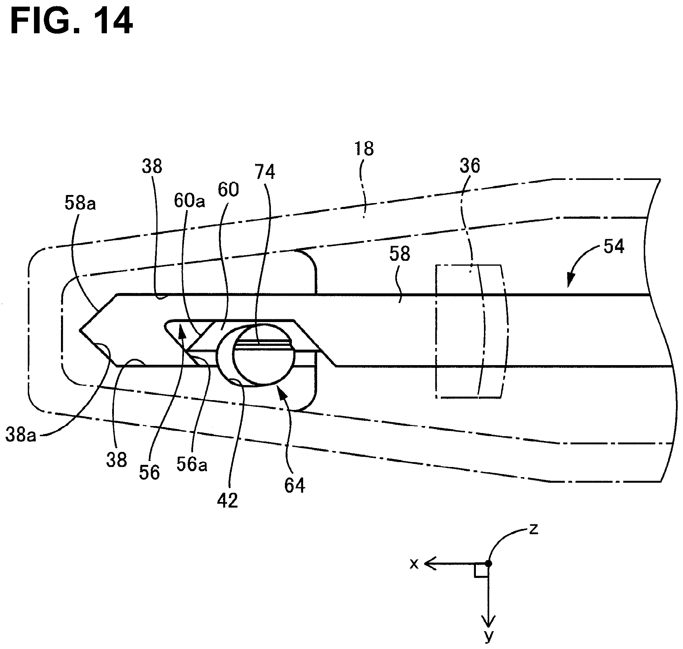

FIG. 14 illustrates a relationship between the resetting thread hook 54 and the thread feed rod 64 when the resetting thread hook 54 has reached its farthest position on the distal end side of the ligation device 10, a distal end portion of the notch 56 functioning as the thread hook portion of the resetting thread hook 54 is positioned further to the distal end side of the second jaw portion 18 in the long side direction than the intersecting position of the first thread-hook passage hole 38 and the thread feed rod passage hole 42, and the thread feed rod 64 is in its most protruding position where the thread feed rod 64 protrudes the furthest from the thread feed rod passage hole 42 in the z-direction. In this state, the thread engagement portion 74 formed at the distal end portion of the thread feed rod 64 is located downstream of the thread feed rod passage hole 42 in the z-direction as the first short side direction, that is, at a position apart from the first jaw portion 12 in the z-direction, and is located at a position overlapping with the first thread-hook passage hole 38 in the y-direction as the second short side direction. The recessed groove 76 of the thread feed rod 64 is positioned to overlap the first thread-hook passage hole 38. Thus, the thread T engaged with the thread engagement portion 74 formed at the distal end of the thread feed rod 64 is hooked on the notch 56 functioning as the thread hook portion of the resetting thread hook 54.

In FIG. 14, similar to the distal end surface 46d of the tubular body 46, a distal end of the tubular body 58 has a V-shaped distal end surface 58a including a pair of inclined surfaces, where a central portion of the distal end surface 58a is closer to the distal end in plan view on the x-y plane. Thus, the resetting thread hook 54 is moved forward to the distal end to contact the distal end surface 58a formed at the distal end of the tubular body 58 with the distal end bottom 38a of the first thread-hook passage hole 38, whereby a rotational phase around an axis of the resetting thread hook 54 is aligned.

For example, as illustrated in FIGS. 5 and 6, the oscillation portion 30 of the main body 14 has the second thread-hook passage hole 80 that is linearly continuous with the first thread-hook passage hole 38 when the second jaw portion 18 is closed, and a second cutter 82 that cuts the thread T extending in the second thread-hook passage hole 80. The second cutter 82 includes a disc-shaped second cutter base 82a supported to be rotatable about a fourth rotation axis C4 parallel to the z-direction in the oscillation portion 30, and a second cutter blade 82b standing on the second cutter base 82a. The second cutter base 82a is operated to rotate via a pair of cutter operation wires 78a and 78b fixed to the second cutter base 82a. Each of the cutter operation wires 78a and 78b extends through a corresponding one of a pair of second cutter operation wire passage holes 78 opened between the intermediate main body portion 22 and the lower main body portion 20 on the proximal end of the ligation device 10 illustrated in FIG. 4.

The retaining cylinder 26 of the main body 14 has the third thread-hook passage hole 84 and a fourth thread-hook passage hole 86 between the intermediate main body portion 22 and the upper main body portion 24 disposed one above another. The third thread-hook passage hole 84 and the fourth thread-hook passage hole 86 are linearly connected to the second thread-hook passage hole 80 when the oscillation portion 30 is positioned on the center line of the main body 14. A fifth thread-hook passage hole 88 parallel to the fourth thread-hook passage hole 86 is branched from between the third thread-hook passage hole 84 and the fourth thread-hook passage hole 86, and is defined in the intermediate main body portion 22. The fourth thread-hook passage hole 86 opens between the intermediate main body portion 22 and the upper main body portion 24 at the center of the proximal end of the ligation device 10 illustrated in FIG. 4 in the y-direction. The ligation thread hook 34 is inserted into the fourth thread-hook passage hole 86. The fifth thread-hook passage hole 88 opens in the intermediate main body portion 22 at the center of the proximal end of the ligation device 10 illustrated in FIG. 4 in the y-direction. The resetting thread hook 54 is inserted into the fifth thread-hook passage hole 88.

The ligation thread hook 34 passed through the fourth thread-hook passage hole 86 and the resetting thread hook 54 passed through the fifth thread-hook passage hole 88 are selectively inserted into the first thread-hook passage hole 38, the second thread-hook passage hole 80, and the third thread-hook passage hole 84.

The ligation thread hook 34 is moved between a forward position in the first thread-hook passage hole 38 and a retracted position in the fourth thread-hook passage hole 86. The resetting thread hook 54 is moved between a forward position in the first thread-hook passage hole 38 and a retracted position in the fifth thread-hook passage hole 88. At the retracted position in the fifth thread-hook passage hole 88, the resetting thread hook 54 is at a first position where the resetting thread hook 54 extends in parallel with the ligation thread hook 34 extends through the first thread-hook passage hole 38 and the fourth thread-hook passage hole 86. At the forward position, that is, at a position in the first thread-hook passage hole 38, the resetting thread hook 54 is at a second position where the resetting thread hook 54 is coaxial with the ligation thread hook 34 in the fourth thread-hook passage hole 86. Thus, the thread T can be reset without increasing the size of the ligation device 10.

The thread loop forming portion 90 for forming two thread loops including a first thread loop L1 and a second thread loop L2 in the thread T in the third thread-hook passage hole 84 is disposed in the retaining cylinder 26 of the main body 14. The thread loop forming portion 90 includes a first thread loop shaft 92 for forming the first thread loop L1 and a second thread loop shaft 94 for forming the second thread loop L2. The first thread loop shaft 92 and the second thread loop shaft 94 are accommodated in the intermediate main body portion 22 of the main body 14 so as to be rotatable about the fifth rotation axis C5 and the sixth rotation axis C6, respectively, that intersect the moving paths the ligation thread hook 34 and the resetting thread hook 54 that moves in the third thread-hook passage hole 84 and are parallel to the z-direction that is the first short side direction.

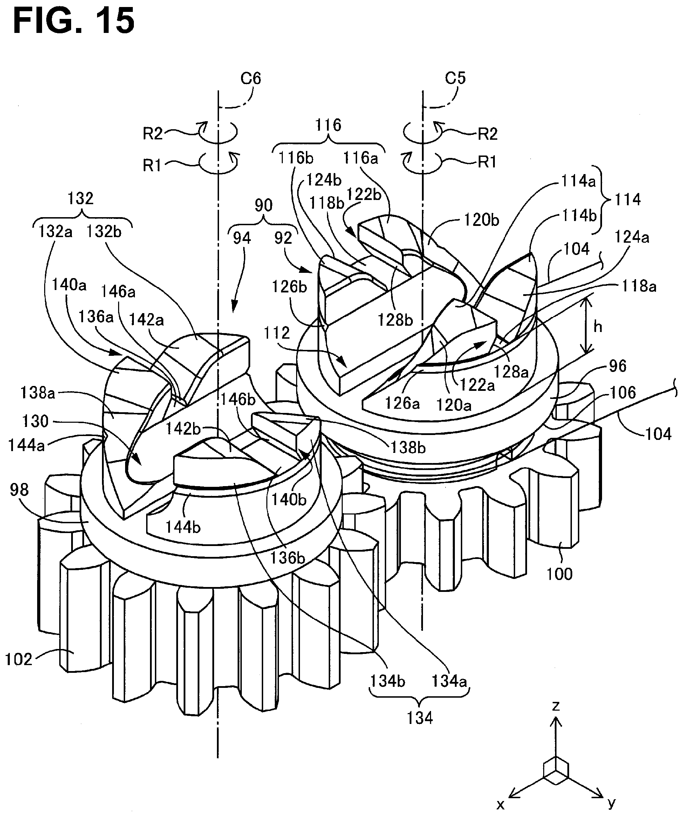

FIG. 15 is an enlarged perspective view of the first thread loop shaft 92 and the second thread loop shaft 94. The first thread loop shaft 92 and the second thread loop shaft 94 have a cylindrical main body portion 96 and a cylindrical main body portion 98, respectively, at their one end portions farther from the third thread-hook passage hole 84 than their other end portions. The first thread loop shaft 92 and the second thread loop shaft 94 include a gear 100 and a gear 102 on an outer peripheral portion of the main body portion 96 and an outer peripheral portion of the main body portion 98, respectively. The gear 100 and the gear 102 are in mesh with each other. The first thread loop shaft 92 and the second thread loop shaft 94 are rotated in opposite directions relative to each other.

The cylindrical main body portion 96 of the first thread loop shaft 92 has a groove 106 around which the thread loop shaft operation wire 104 is wound. End portions of the thread loop shaft operation wire 104 are led out from a pair of thread loop shaft operation wire passage holes 108 and 110 that are opened at a central portion in the z-direction and are apart from each other in the y-direction on the proximal end of the ligation device 10 illustrated in FIG. 4. In response to the end portions of the thread loop shaft operation wire 104 led out from the thread loop shaft operation wire passage holes 108 and 110, respectively, being operated, the first thread loop shaft 92 is rotated and the second thread loop shaft 94 is rotated in the opposite direction by the same angle as the first thread loop shaft 92. In FIG. 15, in plan view, a counterclockwise direction of the first thread loop shaft 92 and a clockwise direction of the second thread loop shaft 94 correspond to a second rotation direction R2, that is, a loop forming rotation direction, and a rotation direction opposite thereto corresponds to a first rotation direction R1, that is, a non-loop-forming rotation direction.

The main body portion 96 of the first thread loop shaft 92 includes a pair of vertical walls 114 and 116 facing each other across a rod guide groove 112 that allows the ligation thread hook 34 or the resetting thread hook 54 moving in the third thread-hook passage hole 84 to pass through. The vertical walls 114 and 116 extend in the first short side direction, that is, in the z-direction. The rod guide groove 112 guides the ligation thread hook 34 or the resetting thread hook 54, thereby functioning as a part of the third thread-hook passage hole 84. The moving path of the ligation thread hook 34 and the moving path of the resetting thread hook 54 are coaxial with each other at a position where the moving path of the ligation thread hook 34 and the moving path of the resetting thread hook 54 intersect with the fifth rotation axis C5 and the sixth rotation axis C6. The pair of vertical walls 114 and 116 are selectively brought into a rotational position state where the vertical walls 114 and 116 are aligned with the moving path of the ligation thread hook 34 or the moving path of the resetting thread hook 54 both moving in the third thread-hook passage hole 84 in accordance with rotation of the first thread loop shaft 92 and another rotational position state where the vertical walls 114 and 116 are shifted from the moving paths. Thus, the thread T is set in the rod guide groove 112 by the backward movement of the resetting thread hook 54, and the ligation thread hook 34 is moved forward through the rod guide groove 112 so that the ligation thread hook 34 passes through the first thread loop L1 and the second thread loop L2 formed around the first thread loop shaft 92 and the second thread loop shaft 94, respectively, by rotation of the first thread loop shaft 92 and the second thread loop shaft 94, whereby the thread T for forming a knot M is intertwined.

The vertical walls 114 and 116 has radial thread grooves 118a and 118b, respectively, functioning as thread guide grooves, at their top portions. The radial thread grooves 118a and 118b extend in respective directions intersecting the rod guide groove 112 around the fifth rotation axis C5, preferably in a direction orthogonal the rod guide groove 112. The radial thread grooves 118a and 118b each have a bottom that is located higher than a bottom of the rod guide groove 112 by a predetermined height amount h in the z-direction. The height amount h is preferably set to be equal to a height that is a distance from the bottom of the rod guide groove 102 to the center of the knot pusher 50 that is the outer tubular member of the ligation thread hook 34 located in the rod guide groove 102.

The vertical wall 114 includes a pair of vertical wall portions 114a and 114b divided by the radial thread groove 118, and the vertical wall 116 includes a pair of vertical wall portions 116a and 116b divided by the radial thread groove 118. The first vertical wall portions 114a and 116a are disposed at a distal end portion of the first thread loop shaft 92 in a first rotation direction R1. The first vertical wall portions 114a and 116a have first thread guide inclined surfaces 120a and 120b, respectively, that extend in a direction away from the bottom of the rod guide groove 112 in the z-direction as the first thread guide inclined surfaces 120a and 120b extend in a second rotation direction R2 of the first thread loop shaft 92. The first thread guide inclined surfaces 120a and 120b guide the thread T in the rod guide groove 112 into the radial thread grooves 118 by moving the thread T in a direction away from the bottom of the rod guide groove 112 in accordance with the rotation of the first thread loop shaft 92 in the first rotation direction R1.

The first vertical wall portions 114a and 116a disposed at the distal end portion of the first thread loop shaft 92 in the first rotation direction R1 include thread engagement corner portions 122a and 122b, respectively, each of which includes a ridgeline as a line of intersection between an inner wall of the radial thread groove 118 and an outer peripheral surface of the first vertical wall 114a or 116a and forms an acute angle slightly smaller than a right angle when viewed in a fifth rotation axis C5 direction, that is, in the z-direction. A tangent line of the outer peripheral surfaces of the first vertical wall portions 114a or 116a passing through the ridge line is inclined as a tangent line at a position shifted by a width of the radial thread groove 118 with respect to a line orthogonal to the long side direction of the radial thread groove 118. Thus, an angle formed by the first vertical wall portion 114a or 116a and an inner peripheral surface of the radial thread groove 118 is an acute angle slightly smaller than a right angle. As the first thread loop shaft 92 rotates in the second rotation direction R2, the thread engagement corner portions 122a and 122b catch the thread T in the radial thread grooves 118a and 118b to form a first thread loop L1 around the fifth rotation axis C5.

Of the pair of vertical wall portions 114a and 114b, and the pair of vertical wall portions 116a and 116b, unlike the first vertical wall potions 114a and 116a, the second vertical wall portions 114b and 116b are disposed at a distal end portion of the first thread loop shaft 92 in the second rotation direction R2. The second vertical wall portions 114b and 116b have second thread guide inclined surfaces 124a and 124b, respectively, that extend in a direction away from the bottom of the rod guide groove 112 in the z-direction as the second thread guide inclined surfaces 124a and 124b extend in the second rotation direction R2 of the first thread loop shaft 92. The second thread guide inclined surfaces 124a and 124b move the thread T in a direction away from the radial thread grooves 118a and 118b in accordance with the rotation of the first thread loop shaft 92 in the first rotation direction R1.

The vertical walls 114 and 116 have, on their outer peripheral surfaces, circumferential thread grooves 126a and 126b, respectively, which is at the same height as the bottoms of the radial thread grooves 118a and 118b, respectively, in the fifth rotation axis C5 direction. Each of the circumferential thread grooves 126a and 126b continuously extends in a circumferential direction for positioning the thread T. The circumferential thread grooves 126a and 126b enable the first thread loop L1 to be stably wound around the outer peripheral surfaces of the vertical walls 114 and 116.

The radial thread grooves 118 has thread movement inclined surfaces 128a and 128b at inner walls of the first vertical wall portions 114a and 116a, respectively, of inner wall of the radial thread grooves 118. The thread movement inclined surfaces 128a and 128b extend in the first rotation direction R1 that is the non-loop-forming rotation direction as the thread movement inclined surfaces 128a and 128b extend toward the bottoms of the radial thread grooves 118. Thus, in response to the first thread loop shaft 92 being rotated in the second rotation direction R2, the thread T in the radial thread grooves 118a and 118b is moved toward the bottom of the radial thread grooves 118 and guided toward the circumferential thread grooves 126a and 126b, whereby the thread T is readily engaged in the circumferential thread grooves 126a and 126b.

Similar to the main body portion 96 of the first thread loop shaft 92, the main body portion 98 of the second thread loop shaft 94 includes vertical walls 132 and 134 disposed on opposite sides of the rod guide groove 130, radial thread grooves 136a and 136b, vertical wall portions 132a and 132b divided by the radial thread groove 136a, vertical wall portions 134a, 134b divided by the radial thread groove 136b, first thread guide inclined surfaces 138a and 138b, thread engagement corner portions 140a and 140b, second thread guide inclined surfaces 142a and 142b, circumferential thread grooves 144a and 144b, and thread movement inclined surfaces 146a and 146b.

### (Ligating Operation by Ligation Device)

Steps of a ligating operation by the ligation device 10 configured as described above will be described below with reference to FIGS. 16 to 44.

FIG. 16 illustrates a ligation object holding step P1 in which a ligation object 32 is placed and held between the lower jaw portion 12 and the upper jaw portion 18 by opening the upper jaw portion 18 using the ligation device 10 in which the thread T is reset. In the ligation device 10 having this state, the thread feed rod 64 is accommodated in the thread feed rod accommodating hole 66. Further, the ligation thread hook 34 is located in the second thread-hook passage hole 80 by the forward movement, and the resetting thread hook 54 is located in the fifth thread-hook passage hole 88. The thread T drawn from the bobbin 62 is held by the resetting thread hook 54 located in the fifth thread-hook passage hole 88 via the hook pin 67, the thread engagement portion 74 of the thread feed rod 64, the first thread-hook passage hole 38, the second thread-hook passage hole 80, and the third thread-hook passage hole 84. In addition, a first loop L1 and a second loop L2 formed by the thread loop forming portion 90 using the thread T are wound around the first thread loop shaft 92 and the second thread loop shaft 94, respectively, at the position corresponding to the thread loop forming portion 90 of the ligation thread hook 34 that has been moved forward. For easy understanding, FIGS. 16 to 23 illustrates a state in which the first loop L1 and the second loop L2 are removed from the first thread loop shaft 92 and the second thread loop shaft 94.

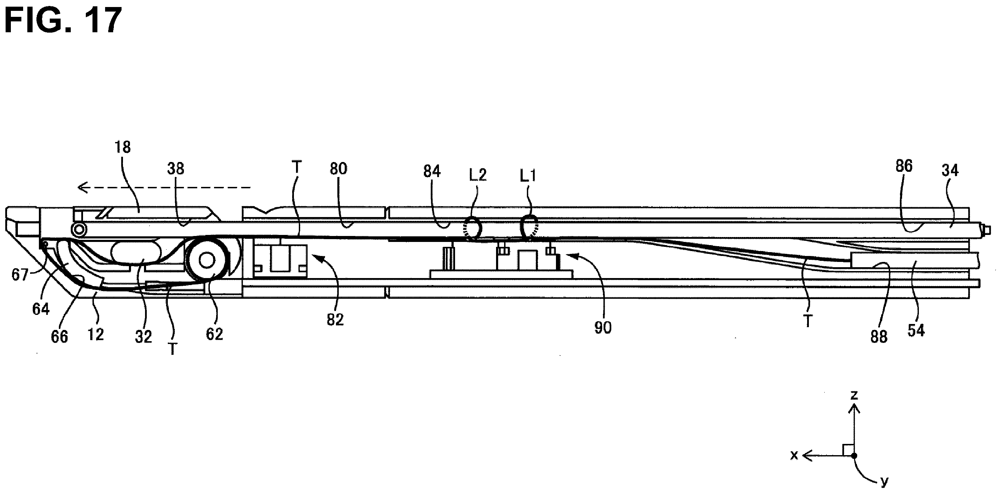

In a knot pusher forward moving step P2 illustrated in FIG. 17, after the upper jaw portion 18 is closed from the state of the ligation object holding step P1 of FIG. 16, the ligation thread hook 34 is moved forward together with the knot pusher 50 to a position just before the opening of the thread feed rod accommodating hole 66 in the first thread-hook passage hole 38. In FIG. 17 and the subsequent drawings, a broken line arrow indicates a moving direction and a moving distance of a specific component, and a solid line arrow indicates a moving direction of the thread T caused by the movement of the specific component.

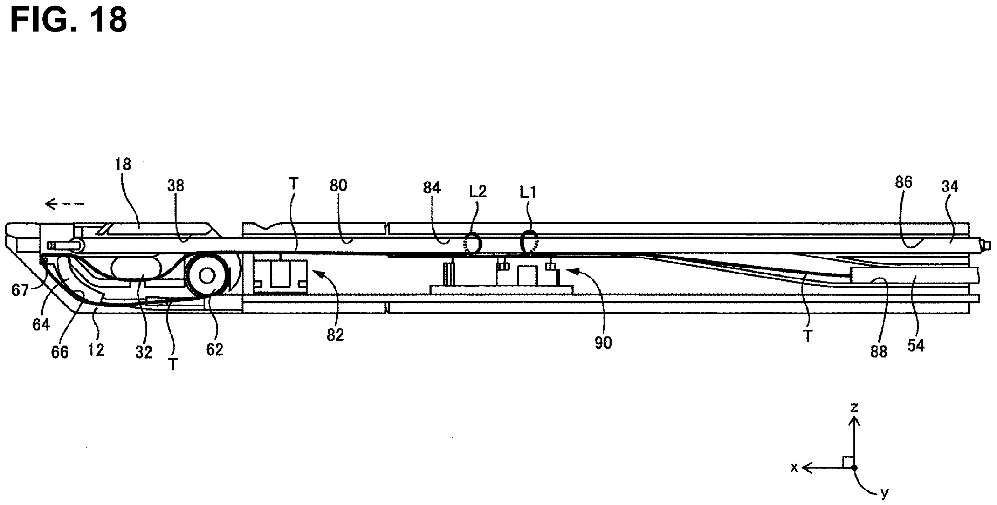

Next, in a ligation thread hook protruding step P3 illustrated in FIG. 18, the tubular body 46 and the thread hook holding member 48 are caused to protrude from the knot pusher 50 of the ligation thread hook 34 to the distal end of the first thread-hook passage hole 38.

Next, in a thread feed rod protruding step P4 illustrated in FIG. 19, the thread feed rod 64 is moved upward in the z-direction until the thread feed rod 64 is exposed from the thread feed rod passage hole 42 defined in the second jaw portion 18.

As a result, the thread T hooked on the thread engagement portion 74 of the thread feed rod 64 is lifted toward the second jaw portion 18.

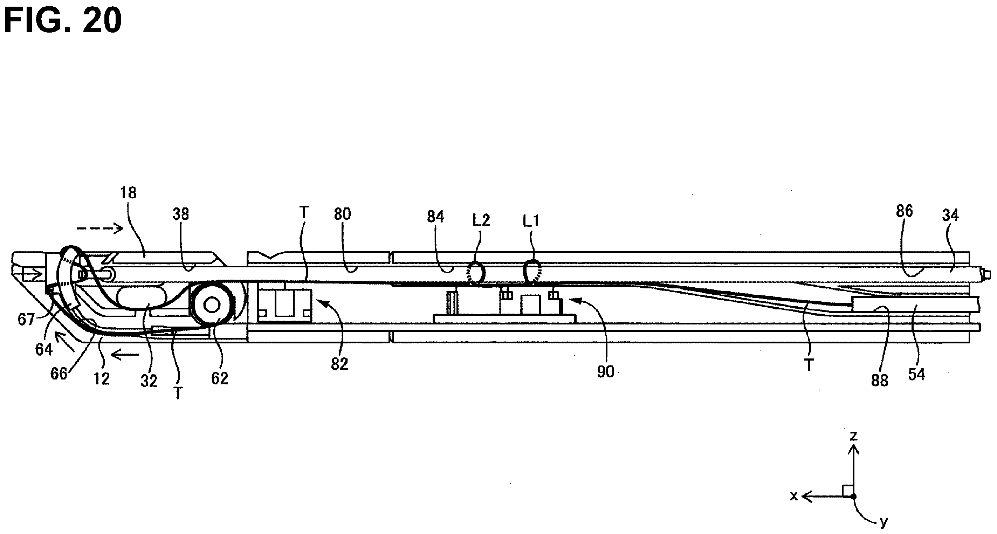

Next, in a thread holding step P5 illustrated in FIG. 20, the thread hook holding member 48 is reciprocated with respect to the tubular body 46 of the ligation thread hook 34 to insert the thread T between the hooking projection 46a and the thread hook holding member 48, thereby loosely holding the thread T therebetween. Then, the ligation thread hook 34 is moved backward slightly until the ligation thread hook 34 is spaced away from the thread feed rod 64.

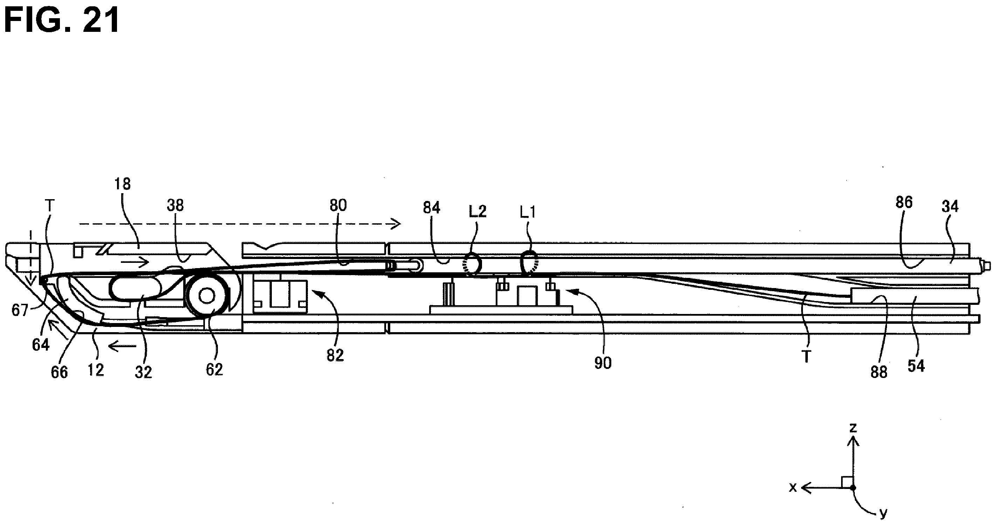

Next, in a knot pusher backward moving step P6 illustrated in FIG. 21, the thread feed rod 64 is moved into the thread feed rod accommodating hole 66, and the ligation thread hook 34 is moved backward together with the knot pusher 50 so that the distal end of the ligation thread hook 34 is located at the distal end position of the third thread-hook passage hole 84.

Next, in a first thread cutting step P7 illustrated in FIG. 22, the ligation thread hook 34 is moved backward slightly in a state in which the thread T is securely held by the thread hook holding member 48 of the ligation thread hook 34, and then the thread feed rod 64 is caused to protrude to an uppermost position in the z-direction through the thread feed rod passage hole 42 defined in the second jaw portion 18. As a result, the thread T to which tension has been applied by the backward movement of the ligation thread hook 34 contacts the first cutter 36 and is thus cut by the first cutter 36. Thus, the thread T is separated into a distal thread T1 and a proximal thread T2 with respect to the ligation object 32. The distal thread T1 is held by the ligation thread hook 34, and the proximal thread T2 is held by the resetting thread hook 54. In this state, the ligation object 32 is wound by the distal thread T1 and the proximal thread T2.

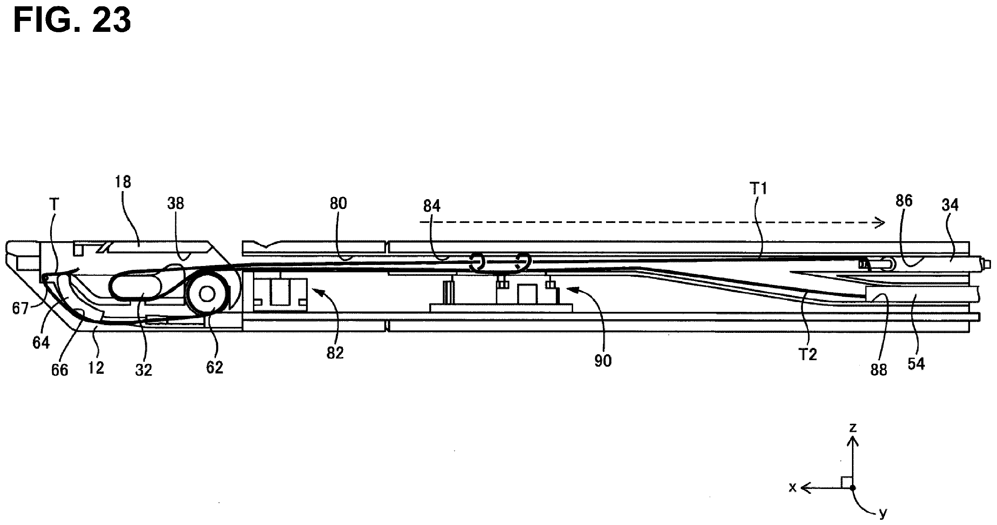

In a thread drawing step P8 illustrated in FIG. 23, after the thread feed rod 64 is accommodated in the thread feed rod accommodating hole 66, the thread hook holding member 48 of the ligation thread hook 34 and the knot pusher 50 are moved back to the proximal end portion of the ligation device 10, that is, to the fourth thread-hook passage hole 86 while the thread T1 is loosely held by the thread hook holding member 48 of the ligation thread hook 34. As a result, the thread T1 is drawn through the two loops that are the second thread loop L2 and the first thread loop L1.

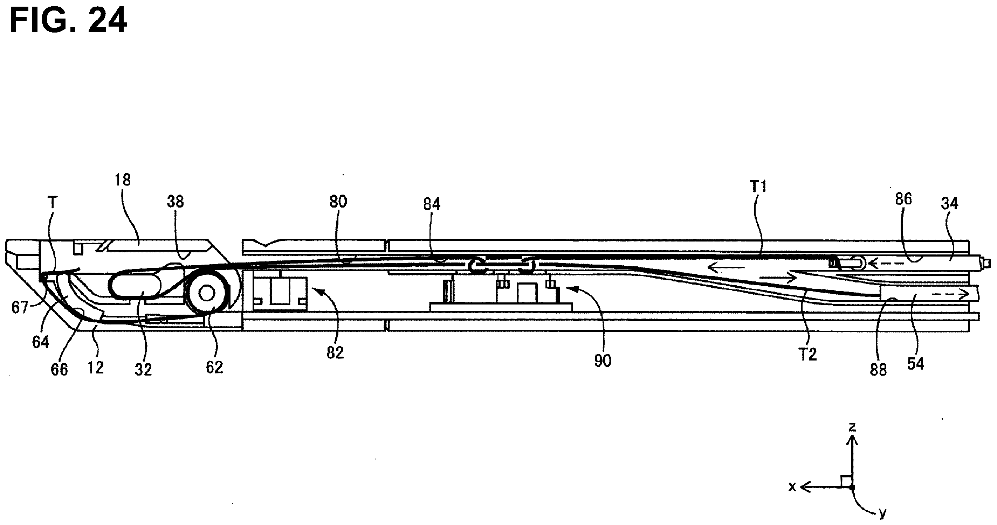

Then, in a thread loop removing step P9 illustrated in FIG. 24, the first thread loop shaft 92 and the second thread loop shaft 94 are rotated in the first rotation direction R1, that is, the non-loop-forming rotation direction with the thread T1 securely held by the ligation thread hook 34. In response to this, the first thread loop L1 wound around the first thread loop shaft 92 and the second thread loop L2 wound around the second thread loop shaft 94 are removed from the first thread loop shaft 92 and the second thread loop shaft 94, respectively, by the action of the second thread guide inclined surfaces 124a and 124b of the first thread loop shaft 92 and the second thread guide inclined surfaces 142a and 142b of the second thread loop shaft 94. In this step, in order to prevent the thread T1 and the thread T2 from being loosened, the ligation thread hook 34 is slightly moved forward and the resetting thread hook 54 is moved backward slightly to apply tensions to the thread T1 and the thread T2.

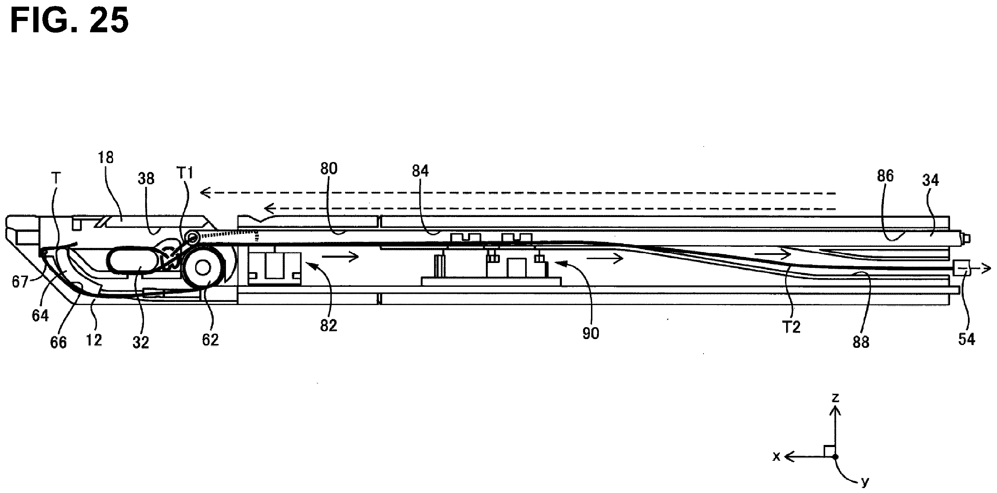

Next, in a thread loop moving step P10 illustrated in FIG. 25, while the thread hook holding member 48 of the ligation thread hook 34 is moved forward to the vicinity of the second cutter 82 with tension being applied to the thread T2 by a slight backward movement of the resetting thread hook 54, the knot pusher 50 of the ligation thread hook 34 is moved forward to the vicinity of the ligation object 32, thereby moving the first thread loop L1 and the second thread loop L2 to the vicinity of the ligation object 32.

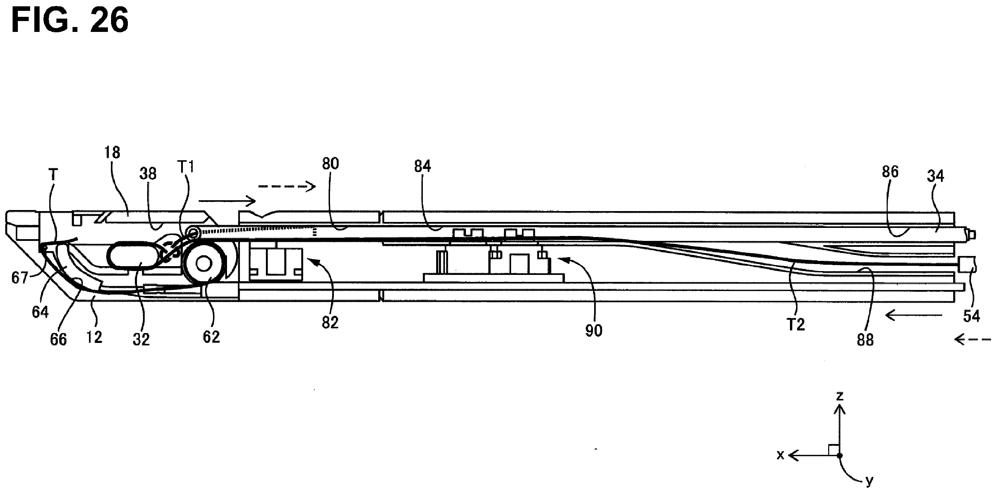

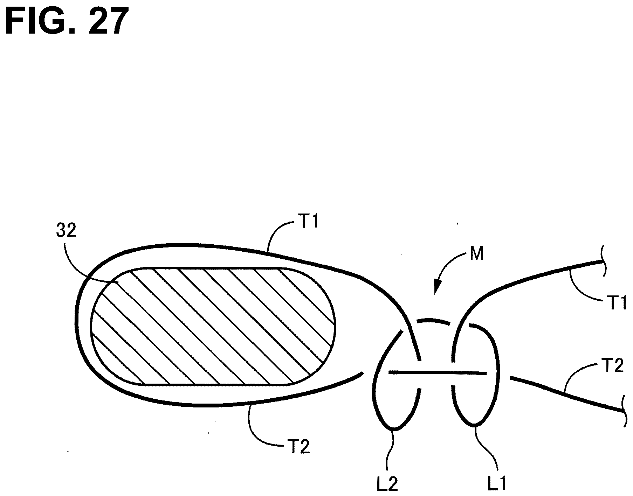

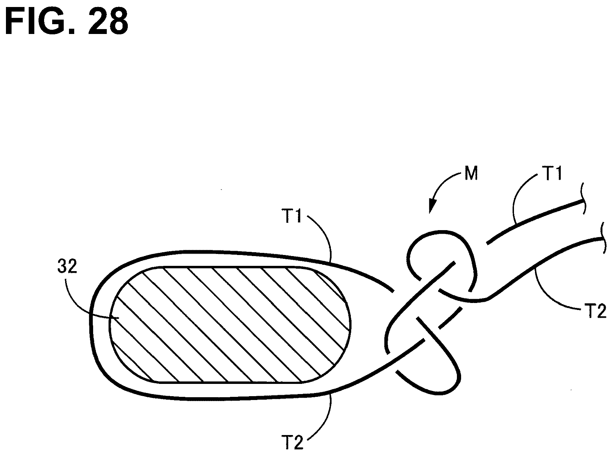

In a knot forming step P11 illustrated in FIG. 26, the resetting thread hook 54 is moved forward and the ligation thread hook 34 is moved backward, thereby shifting (moving) the thread loop L1 closer to the knot pusher 50 of the two thread loops L1 and L2 from the proximal thread T2 to the distal thread T1. As a result, a knot M, for example, a square knot, of FIG. 28 is formed from the state where any knot has not been formed in the thread loop moving step P10 illustrated in FIG. 27. In FIGS. 27 and 28, for easier understanding of a concept of the intertwinement of the distal thread T1 and the proximal thread T2 wound around the ligation object 32, the entanglement of the thread T1 and the thread T2 are illustrated loosely.

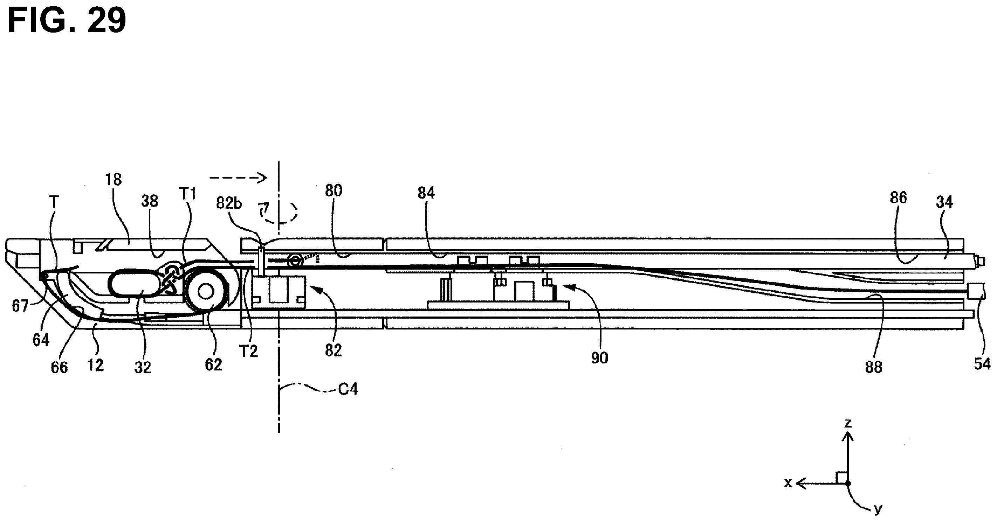

Then, in a second thread cutting step P12 illustrated in FIG. 29, the knot pusher 50 is moved further toward the rear end of the ligation device 10 than the second cutter 82, and the second cutter 82 is operated to rotate about the fourth rotation axis C4 from a retracted position to a cutting position, thereby simultaneously cutting the two threads T1 and T2 extending between the knot M and the ligation thread hook 34 and between the knot M and the resetting thread hook 54, respectively.

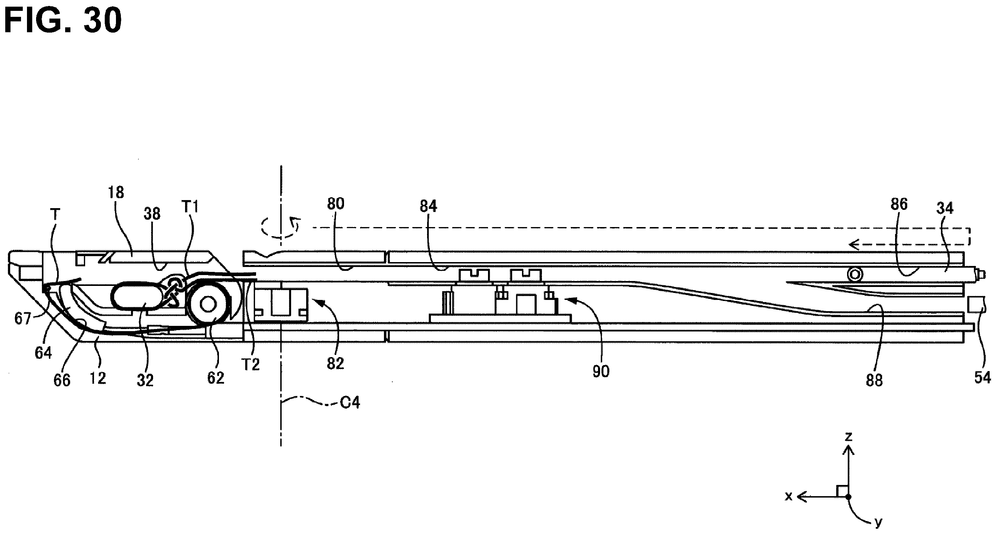

Next, in a thread collecting step P13 illustrated in FIG. 30, the second cutter 82 is operated from the cutting position to the retracted position and the ligation thread hook 34 is pulled out from the ligation device 10 via the rear end of the ligation device 10, whereby a remaining thread is collected from the ligation thread hook 34 and then the knot pusher 50 and the ligation thread hook 34 are inserted into the fourth thread-hook passage hole 86.

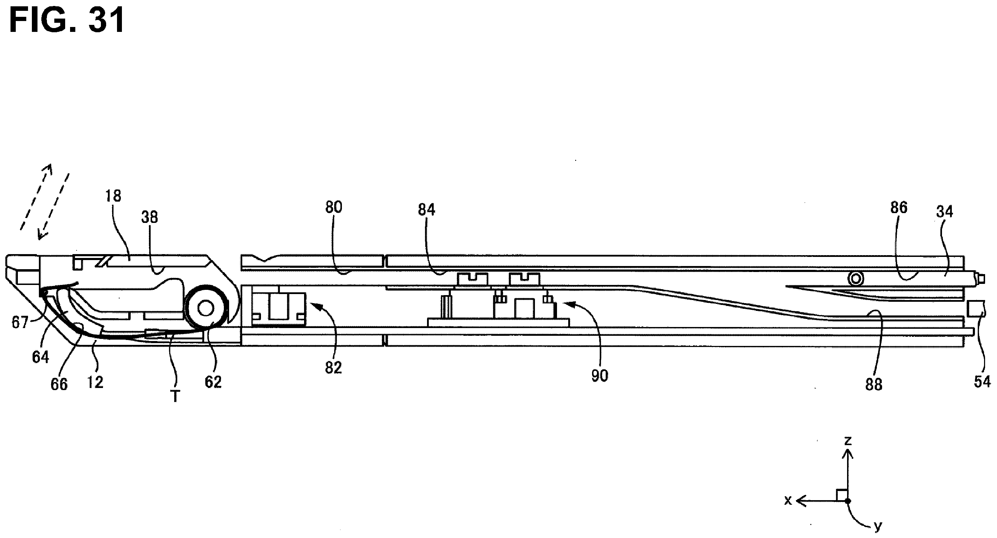

In a ligation object removing step P14 illustrated in FIG. 31, the second jaw portion 18 of the ligation device 10 is opened and closed, whereby the ligation object 32 which has been ligated using the thread T by forming the knot M is removed. FIG. 31 illustrates a state where the second jaw portion 18 is closed after the ligation object 32 is removed.

### (Thread Resetting Operation in Ligation Device)

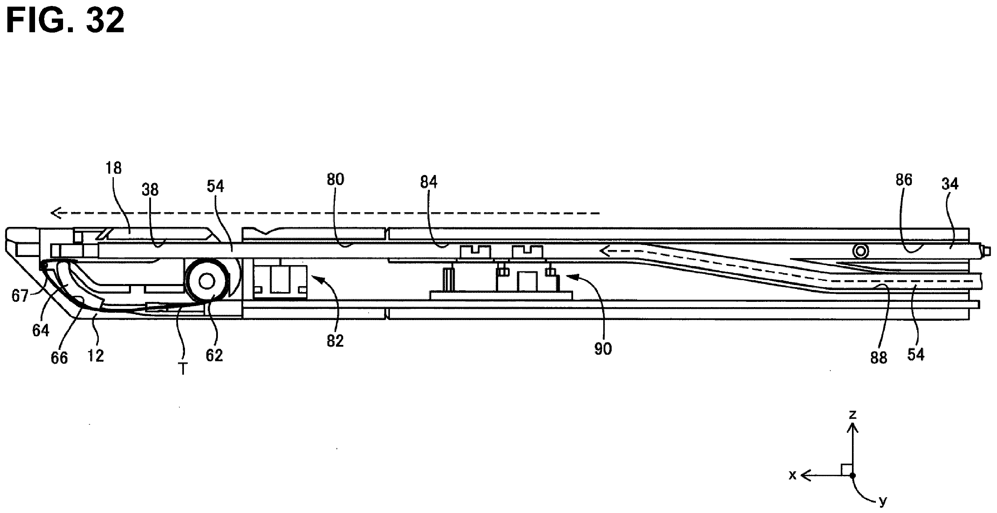

In a resetting thread hook forward moving step P15 illustrated in FIG. 32, in the state illustrated in FIG. 31, the resetting thread hook 54 is inserted via the fifth thread-hook passage hole 88 and is moved forward to the first thread-hook passage hole 38. In this state, the tubular body 58 of the resetting thread hook 54 has reached the distal end of the first thread-hook passage hole 38.

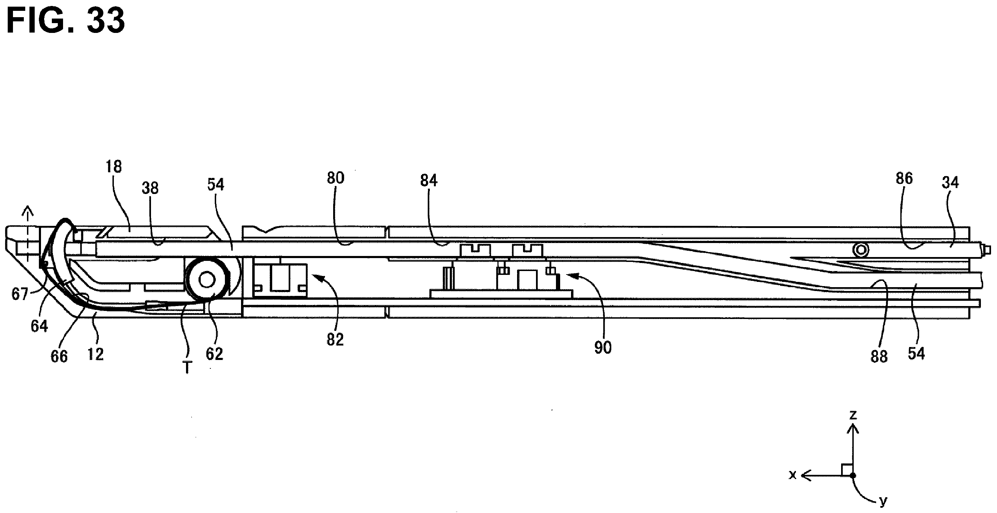

Next, in a thread feed rod protruding step P16 illustrated in FIG. 33, the thread feed rod 64 is moved upward in the z-direction until the thread feed rod 64 reaches the vicinity of the opening of the thread feed rod passage hole 42 defined in the second jaw portion 18.

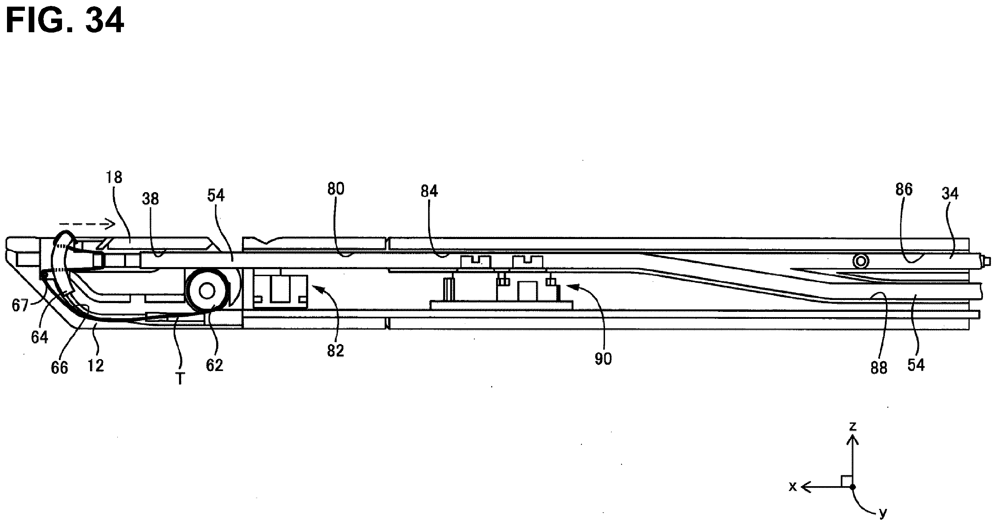

In a thread holding step P17 illustrated in FIG. 34, the thread hook holding member 60 in the tubular body 58 of the resetting thread hook 54 is moved forward to catch and hold the thread T between the notch 56 of the resetting thread hook 54 and the thread hook holding member 60. Thereafter, the resetting thread hook 54 is moved backward slightly.

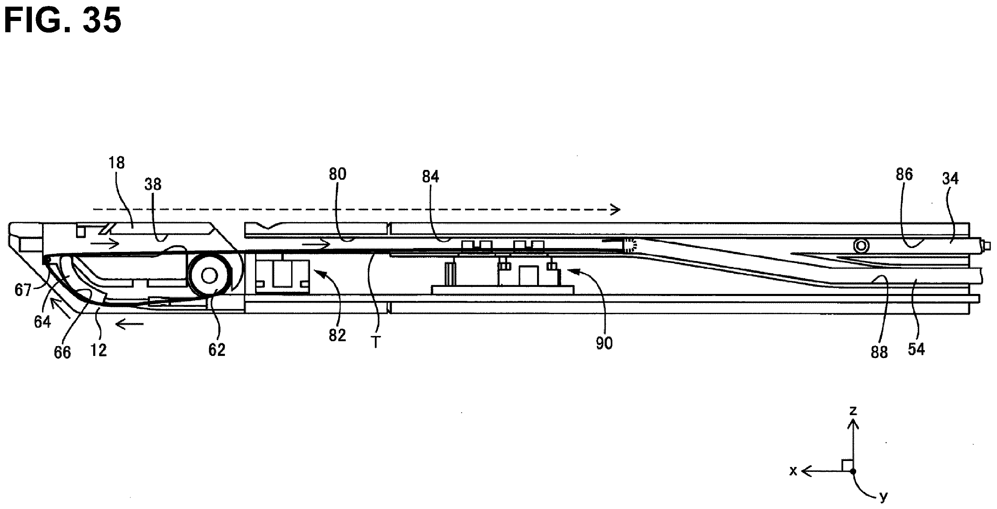

Then, in a rod-guide-groove thread setting step P18 illustrated in FIG. 35, the thread feed rod 64 is lowered and thus accommodated in the thread feed rod accommodating hole 66, and the resetting thread hook 54 is moved backward such that the resetting thread hook 54 is located closer to the proximal end of the ligation device 10 than the thread loop forming portion 90 including the pair of the first thread loop shaft 92 and the second thread loop shaft 94. As a result, the thread T is set to extend through both the rod guide groove 112 of the thread loop shaft 92 and the rod guide groove 130 of the second thread loop shaft 94, where the resetting thread hook 54 was previously located.

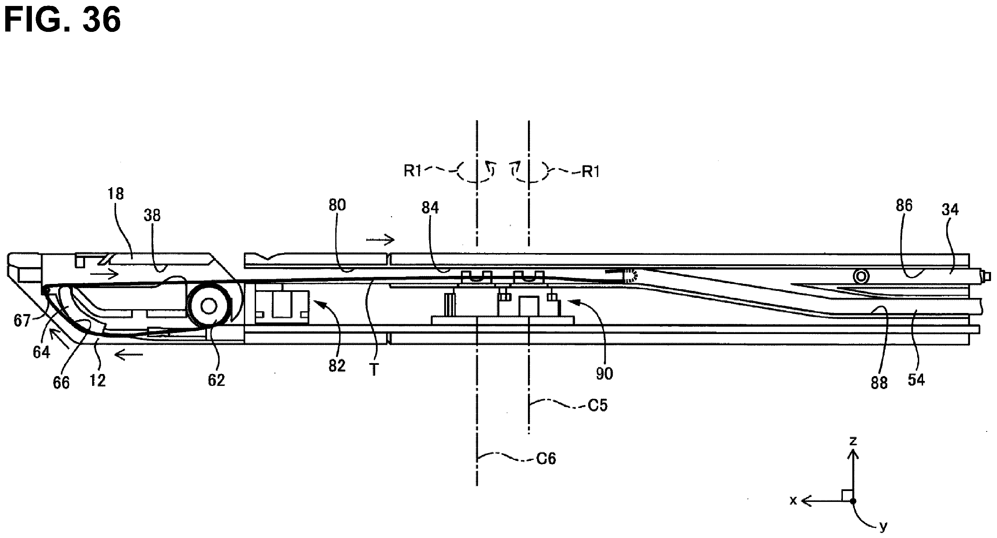

Next, in a thread groove thread setting step P19 illustrated in FIG. 36, the first thread loop shaft 92 and second thread loop shaft 94 are each rotated by 90 degrees in the first rotation direction R1, whereby the thread T extending through the rod guide grooves 112 and 130 is set in the radial thread grooves 118a and 118b extending in direction orthogonal to the rod guide groove 112 and the radial thread grooves 136a and 136b in the direction orthogonal to the rod guide groove 130. In response to the first thread loop shaft 92 and the second thread loop shaft 94 being each rotated by 90 degrees in the first rotation direction R1 with the thread T tensioned to some extent in the x-direction, the thread T is lifted in the z- (upward) direction by the action of the first thread guide inclined surfaces 120a, 120b, 138a, and 138b. In response to the radial thread grooves 118a, 118b, 136a, and 136b reaching the thread T, the thread T comes into the radial thread grooves 118a, 118b, 136a, and 136b, whereby the thread T is positioned into the radial thread grooves 118a, 118b, 136a, and 136b from the rod guide grooves 112 and 130.

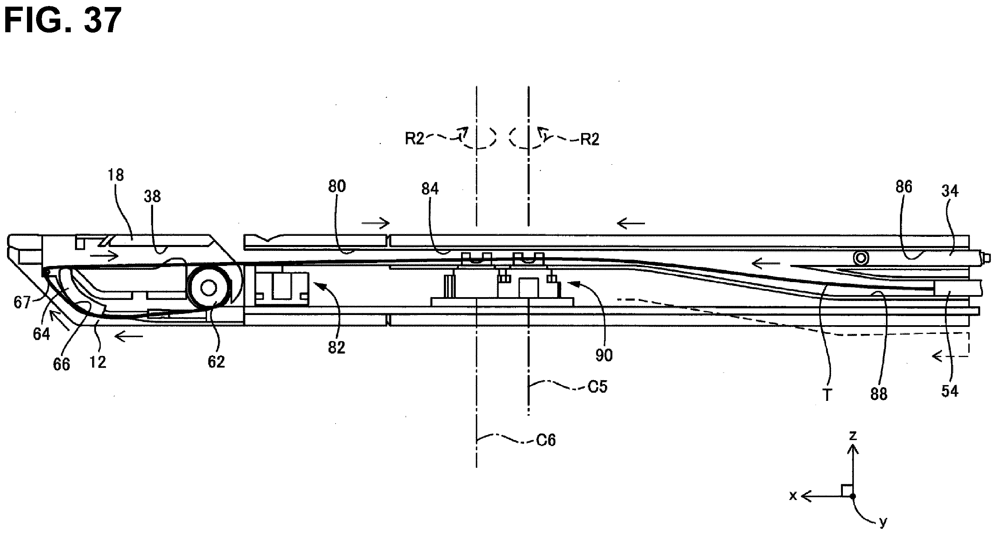

In a thread loop forming step P20 illustrated in FIG. 37, the resetting thread hook 54 is moved backward to the fifth thread-hook passage hole 88. Next, while the resetting thread hook 54 is moved forward, the first thread loop shaft 92 and the second thread loop shaft 94 are each rotated by 270 degrees in the second rotation direction R2 opposite to the first rotation direction R1. As a result, the thread T is wound around each of the first thread loop shaft 92 and the second thread loop shaft 94, thereby forming two thread loops, the first thread loop L1 and the second thread loop L2.

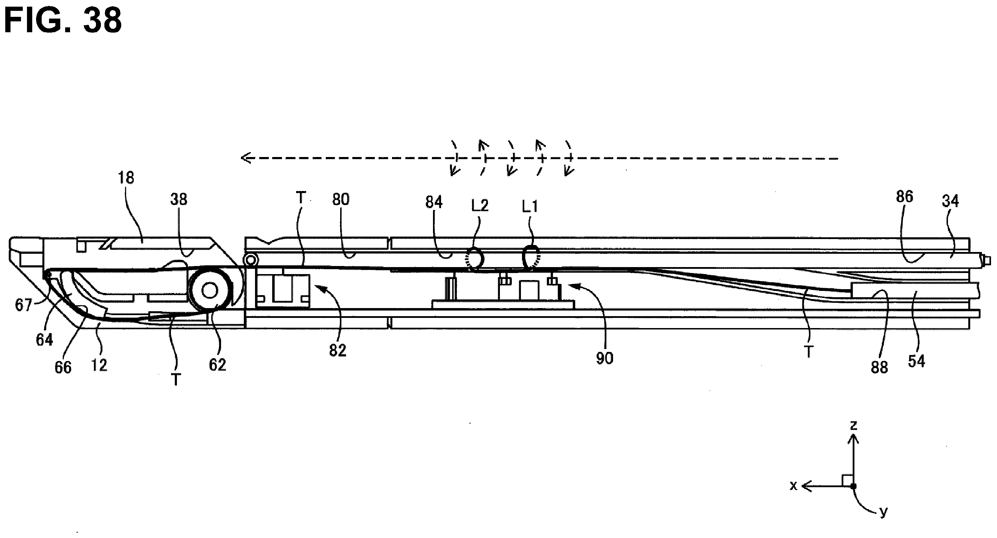

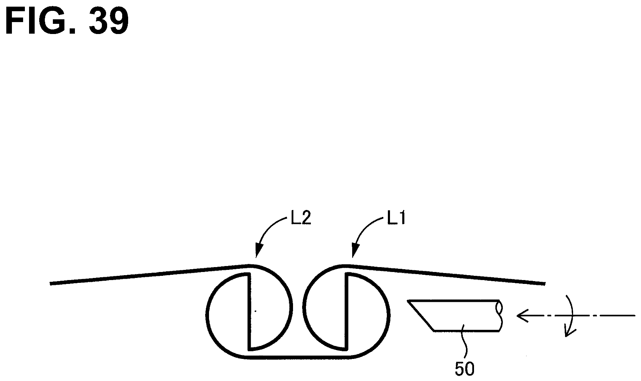

Next, in a thread hook placing step P21 illustrated in FIG. 38, the knot pusher 50 of the ligation thread hook 34 is moved forward while being rotated about the axis and passing through the thread of the first thread loop L1 wound around the first thread loop shaft 92 and the thread of the second thread loop L2 wound around the second thread loop shaft 94 as illustrated in FIGS. 39 to 44.

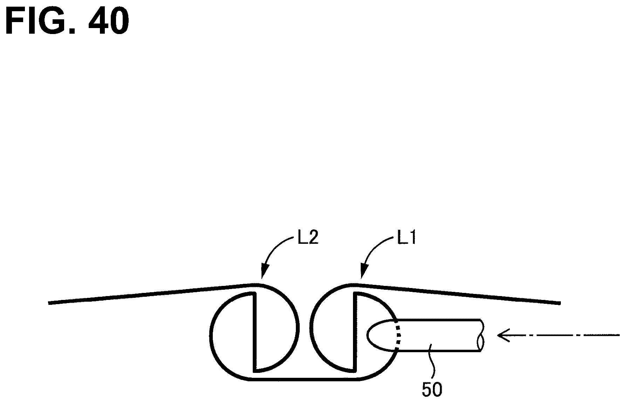

FIG. 39 illustrates a state in which the knot pusher 50 is approaching a proximal-side circumferential thread of the first thread loop L1 located toward the proximal end of the ligation device 10. In a state where the knot pusher 50 crosses the proximal-side circumferential thread of the first thread loop L1, the knot pusher 50 is rotated about the axis so that the inclined end face 50a of the knot pusher 50 faces downward and the knot pusher 50 is caused to pass above the proximal-side circumferential thread of the first thread loop L1 as illustrated in FIG. 40.

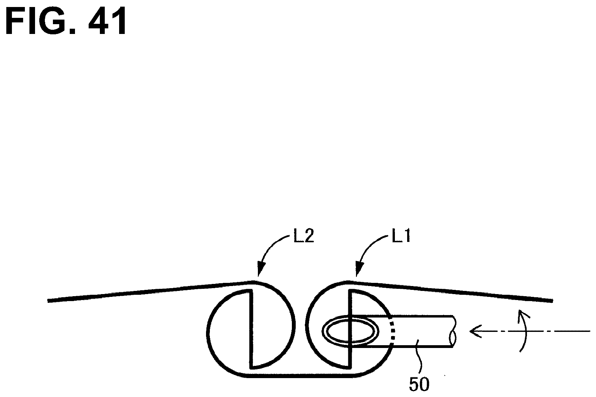

Next, the knot pusher 50 is rotated about the axis so that the inclined end face 50a faces upward, and is moved forward, whereby the knot pusher 50 passes below the radial thread of the first thread loop L1 as illustrated in FIG. 41.

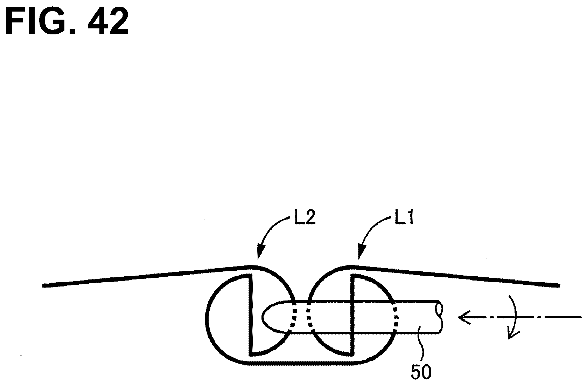

Next, the knot pusher 50 is rotated about the axis so that the inclined end face 50a faces downward, and is moved forward, whereby the knot pusher 50 is caused to pass above the circumferential thread of the first thread loop L1 located toward the proximal end of the ligation device 10 and the radial thread of the second thread loop L2 located toward the proximal end of the ligation device 10 as illustrated in FIG. 42.

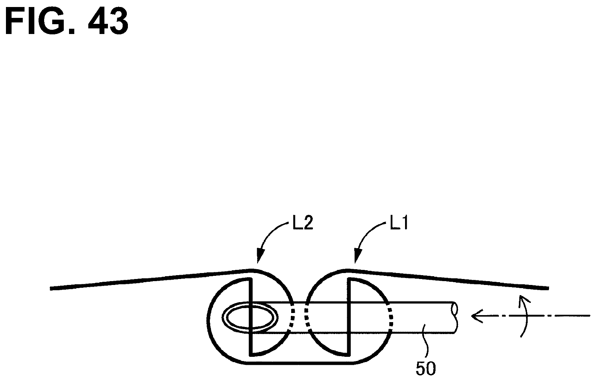

Next, the knot pusher 50 is rotated about the axis so that the inclined end face 50a faces upward, and is moved forward, whereby the knot pusher 50 passes below the radial thread of the second thread loop L2 as illustrated in FIG. 43.

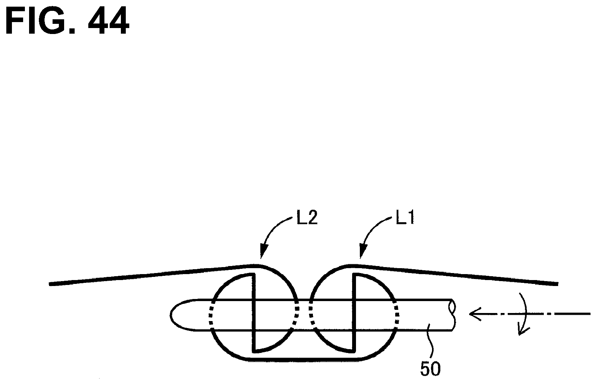

Next, the knot pusher 50 is rotated about the axis so that the inclined end face 50a faces downward, and is moved forward, whereby the knot pusher 50 passes above the circumferential thread of the second thread loop L2 located toward the proximal end of the ligation device 10 as illustrated in FIG. 44.

As described above, as illustrated in FIGS. 39 to 44, the knot pusher 50 is moved forward while being rotated around the axis and passing through the thread of the first thread loop L1 wound around the first thread loop shaft 92 and the thread of the second thread loop L2 wound around the second thread loop shaft 94. As a result, the thread T is reset as illustrated in FIG. 16 and the subsequent drawings.

As described above, the ligation device 10 of the present embodiment includes the columnar main body 14, the first jaw portion 12, the second jaw portion 18, the thread feed rod 64, the ligation thread hook 34, and the resetting thread hook 54. The ligation thread hook 34 and the resetting thread hook 54 are examples of the thread pulling members. The first jaw portion 12 is connected to the one end of the main body 14 in the x-direction. The second jaw portion 18 is connected to the one of the main body 14 in the x-direction so as to be movable between the holding position where the second jaw portion 18 and the first jaw portion 12 hold the ligation object 32 therebetween and the separated position where the second jaw portion 18 is separated from the first jaw portion 12. The thread feed rod 64 has, at its distal end, the thread engagement portion 74 to be engaged with the thread T for ligating the ligation object 32. The thread feed rod 64 is accommodated in the first jaw portion 12. The thread feed rod 64 moves between the retracted position where the thread engagement portion 74 is located inside the first jaw portion 12 and the protruding position where the thread engagement portion 74 protrudes from the first jaw portion 12 when the second jaw portion 18 is located at the holding position. The ligation thread hook 34 and the resetting thread hook 54, which are examples of thread pulling members, are disposed in the main body 14. The ligation thread hook 34 has, at the distal end thereof, the notch 44 that is an example of the thread hook portion for catching the thread T engaged with the thread engagement portion 74. The resetting thread hook 54 has, at the distal end thereof, the notch 56 that is an example of the thread hook portion for catching the thread T engaged with the thread engagement portion 74. The second jaw portion has the first thread-hook passage hole 38 and the thread feed rod passage hole 42. The first thread-hook passage hole 38 extends along the long side direction, and the thread hook portion is inserted the first thread-hook passage hole 38. The thread feed rod passage hole 42 extends along the z-direction that is the direction from the first jaw portion 12 toward the second jaw portion 18 and is the first short side direction intersecting the long side direction, and the thread feed rod 64 is inserted into the thread feed rod passage hole 42. The first thread-hook passage hole 38 and the thread feed rod passage hole 42 at least partially intersect with each other. When the notch 44 or 56 that is an example of the thread hook portion is located closer to the distal end of the second jaw portion 18 in the long side direction than the intersecting position of the first thread-hook passage hole 38 and the thread feed rod passage hole 42 and the thread feed rod 64 is at the protruding position, the thread engagement portion 74 of the thread feed rod 64 is positioned downstream of the first thread-hook passage hole 38 in the z-direction that is the first short side, that is, at a position shifted away from the first jaw portion 12, and the thread engagement portion 74 is at a position overlapping the first thread-hook passage hole 38 in the y-direction that is the second short side direction.

According to the ligation device 10 of the present embodiment, when the notch 44 of the ligation thread hook 34 or the notch 56 of the resetting thread hook 54 is located closer to the distal end of the second jaw portion 18 in the long side direction than the intersection position of the first thread-hook passage hole 38 and the thread feed rod passage hole 42 and the thread feed rod 64 is at the protruding position, the thread engagement portion 74 of the thread feed rod 64 is located further from the first jaw portion 12 than the first thread-hook passage hole 38 in the z-direction as the first short side direction and at the position overlapping the first thread-hook passage hole 38 in the y-direction, that is, the second short side direction. Thus, the thread T can be directly wound around the outer periphery of the ligation object 32 by holding the thread by the thread hook.

According to the ligation device 10 of the present embodiment, the thread feed rod 64 has the recessed groove 76 upstream of the thread engagement portion 74 in the z-direction as the first short side direction and at the position overlapping the thread engagement portion 74 in the y-direction as the second short side direction. When the thread feed rod 64 is at the protruding position, the recessed groove 76 is at the position overlapping the first thread-hook passage hole 38 in the z-direction as the first short side direction. As a result, the thread engagement portion 74 of the thread feed rod 64 can cross the notch 44 of the ligation thread hook 34 that is the example of the thread pulling member in the y-direction as the second short side direction.

According to the ligation device 10 of the present embodiment, the ligation thread hook 34 that is the first thread pulling member has the tubular body 46 having the notch 44 constituting the thread hook portion, and the thread hook holding member 48 that is slidable into the tubular body 46 and catches and holds the thread T between the thread hook holding member 48 and the notch 44. Similarly, the resetting thread hook 54 that is the second thread pulling member has the tubular body 58 having the notch 56 constituting the thread hook portion, and the thread hook holding member 60 that is slidable into the tubular body 58 and catches and holds the thread T between the thread hook holding member 60 and the notch 56. Thus, the thread T caught by the notches 44 and 56 can be held.

According to the ligation device 10 of the present embodiment, the distal end of the thread hook holding member 48 has the inclined surface 48a that is inclined with respect to the x-direction as the long side direction of the ligation device 10 and the y-direction as the second short side direction. Further, the tubular body 46 has the inclined surface 46c that is an opposing surface opposing to the inclined surface 48a, and the hooking projection 46a. The hooking projection 46a linearly projects in the direction away from the distal end of the tubular body 46 from the end of the inclined surface 46c in the x-direction. Thus, the thread T can be held by the notch 44 further reliably.

According to the ligation device 10 of the present embodiment, the inclined surface 46c that is the opposing surface of the tubular body 46 and faces the inclined surface 48a of the distal end of the thread hook holding member 48 is parallel to the inclined surface 48a. As a result, the inclined surface 46c that is the opposing surface of the tubular body 46, and the inclined surface 48a at the distal end of the thread hook holding member 48 come into contact with each other, thereby restricting the rotation about the axis of the thread hook holding member 48 with respect to the tubular body 46. Thus, the thread T can be properly held by the notch 44.

According to the ligation device 10 of the present embodiment, the tubular body 46 has the flat portion 46b at its distal end portion that extends between the notch 44 of the tubular body 46 and the distal end. The flat portion 46b is thinner than the inside diameter of the tubular body 46. Thus, the thread T held between the thread hook holding member and the flat portion can be pulled into the knot pusher 50.

According to the ligation device 10 of the present embodiment, the thread engagement portion 74 of the thread feed rod 64 is a groove extending along the x-direction as the long side direction of the ligation device 10. This enables the thread T to be hooked on the thread engagement portion 74 readily.

According to the ligation device 10 of this embodiment, the first cutter 36 for cutting the thread T is fixed to the second jaw portion 18. The first cutter 36 is located between the thread feed rod passage hole 42 and the main body 14 in the long side direction. Thus, projecting the thread feed rod 64 from the first jaw member 18 enables the cutting of the thread T.

According to the ligation device 10 of this embodiment, the first cutter 36 is fixed to the second jaw portion 18. The thread feed rod 64 is movable from the first protruding position to the second protruding position at which the thread engagement portion 74 of the thread feed rod 64 protrudes further from the first protruding position in the z-direction as the first short side direction. The first protruding position is a position where the thread engagement portion 74 protrudes from the thread feed rod passage hole 42 to cause the ligation thread hook 34 to catch and hold the thread T when the second jaw portion 18 is in the holding position. Thus, the thread T can be cut only by moving the thread feed rod 64 without moving the first cutter 36.

According to the ligation device 10 of the present embodiment, the bobbin 62 in which the thread T is stored as the example of a thread storage portion is disposed in the first jaw portion 12. Thus, as compared with a case where the bobbin 62 is located at a position different from the inside of the first jaw portion 12, a distance between the bobbin 62 and the thread feed rod 64 is shorter, thereby reducing waste of the thread T.

According to the ligation device 10 of the present embodiment, the bobbin 62 in which the thread T is stored is positioned between the thread engagement portion 74 of the thread feed rod 64 and the main body 14 in the long side direction of the ligation device 10. This arrangement thus enables reduction of the dimension of the ligation device 10 in the long side direction, thereby reducing the size of the ligation device 10.

According to the ligation device 10 of the present embodiment, the thread storage portion includes the bobbin 62 around which the thread T is wound, and the third rotation axis C3 that is the center of rotation of the bobbin 62 is parallel to the y-direction as the second short side direction. As a result, the thread T can be supplied to the thread feed rod 64 along the long side direction of the ligation device 10 while reducing twist of the thread T.

According to the ligation device 10 of the present embodiment, the main body 14 includes the thread loop forming portion 90 that forms two thread loops in the thread T that has been pulled into the main body 14 by the resetting thread hook 54 that is the example of the thread pulling member. The thread loop forming portion 90 includes the first thread loop shaft 92 and the second thread loop shaft 94 that are the pair of thread loop shafts. The first thread loop shaft 92 and the second thread loop shaft 94 rotate in opposite directions relative to each other about the respective rotation axes that intersect the moving paths the ligation thread hook 34 and the resetting thread hook 54 and are parallel to each other. The first thread loop shaft 92 includes the main body portion 96, the pair of vertical walls 114 and 116, and the rod guide groove 112. The second thread loop shaft 94 includes the main body portion 98, the pair of vertical walls 132 and 134, and the rod guide groove 130. The main body portions 96 and 98 are disposed apart from the moving paths, and rotate around the first thread loop shaft 92 and the second thread loop shaft 94, respectively. The pair of vertical walls 114 and 116 protrudes from the main body portion 96 in the direction parallel to the fifth rotation axis C5. The pair of vertical walls 132 and 134 protrudes from the main body portion 98 in the direction parallel to the sixth rotation axis C6. The pair of vertical walls 114 and 116 and the pair of vertical walls 132 and 134 are respectively movable to the position overlapping with the moving paths and to the position away from the moving paths across the resetting thread hook 54 in accordance with the rotation of the main body portions 96 and 98. The rod guide grooves 112 and 130 guide the movement of the ligation thread hook 34 and the resetting thread hook 54 in the long side direction of the ligation device 10, that is, the x-direction. Thus, the thread T is set in the rod guide grooves 112 and 130 by the backward movement of the ligation thread hook 34 and the resetting thread hook 54, and the ligation thread hook 34 and the resetting thread hook 54 are moved forward through the rod guide grooves 112 and 130 so that the ligation thread hook 34 and the resetting thread hook 54 pass through the first thread loop L1 and the second thread loop L2 formed around the first thread loop shaft 92 and the second thread loop shaft 94, respectively, by the rotation of the first thread loop shaft 92 and the second thread loop shaft 94, whereby the thread T for forming a knot M is intertwined.

According to the ligation device 10 of the present embodiment, the first thread loop shaft 92 and the second thread loop shaft 94 are disposed so as to be rotatable about the fifth rotation axis C5 and the sixth rotation axis C6, respectively, which are parallel to the first short side direction, that is, the z-direction. The first thread loop shaft 92 and the second thread loop shaft 94 include the gear 100 and the gear 102, respectively, which mesh with each other. The first thread loop shaft 92 and the second thread loop shaft 94 are rotated in opposite directions relative to each other. Thus, the knot M using the thread T is formed based on the first thread loop L1 and the second thread loop L2 formed around the fifth rotation axis C5 and the sixth rotation axis C6, respectively.

According to the ligation device 10 of the present embodiment, the first thread loop shaft 92 includes the radial thread grooves 118a and 118b, the first thread guide inclined surfaces 120a and 120b, and the thread engagement corner portions 122a and 122b. The second thread loop shaft 94 includes the radial thread grooves 135a and 136b, the first thread guide inclined surfaces 138a and 138b, and the thread engagement corner portions 140a and 140b. The radial thread grooves 118a and 118b are grooves extending along the direction orthogonal to the rod guide groove 112. The radial thread grooves 135a and 136b are grooves extending along the direction orthogonal to the rod guide groove 130. The first thread guide inclined surfaces 120a, 120b and 138a, 138b are inclined surfaces for moving, into the radial thread grooves 118a, 118b, 135a, and 136, the thread T pulled into the main body 14 by the ligation thread hook 34 in the rod guide grooves 112 and 130, in response to the rotation of the first thread loop shaft 92 and the second thread loop shaft 94 in the non-loop-forming rotation direction R1. As the first thread loop shaft 92 and the second thread loop shaft 94 rotate in the loop forming rotation direction R2 opposite to the non-loop-forming rotation direction R1, the thread engagement corner portions 122a, 122b, 140a, and 140b catch the thread T in the radial thread grooves 118a, 118b, 135a, and 136 to form a first thread loop L1 and a second thread loop L2 around the fifth rotation axis C5 and the sixth rotation axis C6, respectively. Thus, a knot M using the thread T is formed based on the first thread loop L1 and the second thread loop L2 formed around the fifth rotation axis C5 and the sixth rotation axis C6.

According to the ligation device 10 of the present embodiment, the ligation thread hook 34 includes the knot pusher 50 that is an example of the outer tubular member. The tubular body 46 is slidably inserted into the knot pusher 50. The tubular body 46 is enraged therein so that the tubular body 46 can protrude from the distal end of the knot pusher 50 in the x-direction. The knot pusher 50 has the inclined end face 50a at is distal end. Thus, the knot M can be firmly tied.

According to the ligation device 10 of the present embodiment, the thread pulling member includes the resetting thread hook that moves between the first position where the resetting thread hook extends parallel to the ligation thread hook 34 and the second position where the resetting thread hook is coaxial with the ligation thread hook 34. Such a configuration enables the resetting of the thread T without increasing the size of the ligation device 10.

According to the ligation device 10 of the present embodiment, the ligation device 10 includes the oscillation portion 30 that connects the first jaw portion 12 and the second jaw portion 18 to the main body 14. The distal end portion of the second jaw portion 18 is openably and closably connected to the first jaw portion 12 via the first pins 16 extending parallel to the y-direction as the second short side direction. The oscillation portion 30 that is an example of the coupling member is rotatably coupled to the end portion of the tubular main body 14 via the second pin 28 extending parallel to the z-direction as the first short side direction. This configuration thus enables the second jaw portion 18 to be opened and closed with respect to the first jaw portion 12. The second jaw portion 18 and the first jaw portion 12 are connected to the main body 14 so as to oscillate with respect to the tubular main body 14, thereby increasing flexibility in the operation of the second jaw portion 18 and the first jaw portion 12.

The ligation device 10 of the present embodiment includes the columnar main body 14, the first jaw portion 12, the second jaw portion 18, the resetting thread hook 54, and the thread loop forming portion 90. The first jaw portion 12 and the second jaw portion 18 are connected to the one end of the main body 14 in the long side direction and are the example of the holding portion for holding the ligation object 32. The resetting thread hook 54 is the example of the thread pulling member. The resetting thread hook 54 is disposed so as to be movable along the long side direction in the main body 14, and pulls the thread T for ligating the ligation object 32 into the main body 14. The thread loop forming portion 90 forms two thread loops L1 and L2 in the thread T that has been pulled into the main body 14 by the resetting thread hook 54. The thread loop forming section 90 includes the first thread loop shaft 92 and the second thread loop shaft 94 that rotate in opposite directions relative to each other about the fifth rotation axis C5 and the sixth rotation axis C6, respectively. The fifth rotation axis C5 and the sixth rotation axis C6 are parallel to each other and intersect the movement path of the resetting thread hook 54. The first thread loop shaft 92 has the main body portion 96, the pair of vertical walls 114 and 116, and the radial thread grooves 118a and 118b. The radial thread grooves 118a and 118b are examples of the thread grooves. The second thread loop shaft 94 has the main body portion 98, the pair of vertical walls 132 and 134, and the radial thread grooves 136a and 136b. The radial thread grooves 136a and 136b are examples of the thread grooves. The main body portions 96 and 98 are disposed apart from the moving paths, and rotate around the first thread loop shaft 92 and the second thread loop shaft 94, respectively. The pair of vertical walls 114 and 116 protrudes from the main body portion 96 in the direction parallel to the fifth rotation axis C5. The pair of vertical walls 132 and 134 protrudes from the main body portion 98 in the direction parallel to the sixth rotation axis C6. The pair of vertical walls 114 and 116 and the pair of vertical walls 132 and 134 are respectively movable to the position overlapping with the moving paths and to the position away from the moving paths across the resetting thread hook 54 in accordance with the rotation of the main body portions 96 and 98. The radial thread grooves 118a and 118b are defined in the top portions of the vertical walls 114 and 116, respectively, for engaging with the thread T pulled by the resetting thread hook 54. The radial thread grooves 136a and 136b are defined in the top portions of the vertical walls 132 and 134, respectively, for engaging with the thread T pulled by the resetting thread hook 54.

According to the ligation device 10 of the present embodiment, the first thread loop shaft 92 and the second thread loop shaft 94 rotate in opposite directions with respect to each other about the fifth rotation axis C5 and the sixth rotation axis C6, respectively. The first thread loop shaft 92 includes the pair of vertical walls 114 and 116 and the radial thread grooves 118a, and 118b that are defined in the top portions of the vertical walls 114 and 116 and engage with the thread T pulled by the resetting thread hook 54. The second thread loop shaft 94 includes the pair of vertical walls 132 and 134 and the radial thread grooves 136a and 136b that are defined in the top portions of the vertical walls 132 and 134 and engage with the thread T pulled by the resetting thread hook 54. Therefore, the thread T drawn into the radial thread grooves 118a and 118b and the radial thread grooves 136a and 136b by the resetting thread hook 54 is wound around the outer peripheral surfaces of the pair of vertical walls 114 and 116 and the pair of vertical walls 132 and 134 as the first thread loop shaft 92 and the second thread loop shaft 94 rotate in opposite directions with respect to each other about the fifth rotation axis C5 and the sixth rotation axis C6, respectively, thereby forming two thread loops L1 and L2 using the proximal thread T2. The ligation thread hook 34 moves forward toward the distal end through the two thread loops L1 and L2 to catch and hold the distal thread T1 with respect to the ligation object 32. The ligation thread hook 34 also moves backward toward the proximal side of the ligation object 32. Thus, the distal thread T1 is passed through the thread loops L1 and L2 formed by the proximal thread T2, thereby forming a knot M.

According to the ligation device 10 of the present embodiment, the thread pulling member includes the ligation thread hook 34 and the resetting thread hook 54. The ligation thread hook 34 is an example of a first thread pulling rod. The resetting thread hook 54 is an example of a second thread pulling rod. The ligation thread hook 34 pulls the thread T wound around the ligation object 32 into the main body 14. The resetting thread hook 54 pulls the thread T that is to be wound around the ligation object 32 into the main body 14. The moving path of the ligation thread hook 34 and the moving path of the resetting thread hook 54 are coaxial with each other at a position where the moving path of the ligation thread hook 34 and the moving path of the resetting thread hook 54 intersect with the fifth rotation axis C5 and the sixth rotation axis C6 of the first thread loop shaft 92 and the second thread loop shaft 94, respectively. Thus, by providing partial commonality of the moving paths between the ligation thread hook 34 and the resetting thread hook 54, a base of the knot M to be formed in the thread T can be formed in the thread loop forming portion 90 while reducing a size of the ligation device 10.

According to the ligation device 10 of the present embodiment, the vertical wall 114 includes the pair of vertical wall portions 114a and 114b divided by the radial thread groove 118a, and the vertical wall 116 includes the pair of vertical wall portions 116a and 116b divided by the radial thread groove 118b. The vertical wall 132 includes the pair of vertical wall portions 132a and 132b divided by the radial thread groove 136a, and the vertical wall 134 includes the pair of vertical wall portions 134a and 134b divided by the radial thread groove 136b. Of the pair of vertical wall portions 114a and 114b, the pair of vertical wall portions 116a and 116b, the pair of vertical wall portions 132a and 132b, and the pair of vertical wall portions 134a and 134b, the first vertical wall portions 114a, 116a, 132a, and 134a has the first thread guide inclined surfaces 120a, 120b, 138a, and 138b, respectively. The first thread guide inclined surfaces 120a, 120b, 138a, and 138b guide the thread T to the radial thread grooves 118a, 118b, 136a, and 136b, respectively in accordance with the first rotation of the first thread loop shaft 92 and the second thread loop shaft 92 in the non-loop-forming rotation direction R1. Thus, in the first rotation R1, two loops, that is, the first thread loop L1 and the second thread loop L2 for a squire knot can be formed.

According to the ligation device 10 of the present embodiment, f the pair of vertical wall portions 114a and 114b, the pair of vertical wall portions 116a and 116b, the pair of vertical wall portions 132a and 132b, and the pair of vertical wall portions 134a and 134b, the second vertical wall portions 114b, 116b, 132b, and 134b has the second thread guide inclined surfaces 124a and 124b, respectively. The second thread guide inclined surfaces 124a and 124b move the thread T in a direction away from the radial thread grooves in accordance with the first rotation of the first thread loop shaft 92 and the second thread loop shaft 94 in the non-loop-forming rotation direction R1. Thus, the first thread loop L1 and the second thread loop L2 are readily removed from the first thread loop shaft 92 and the second thread loop shaft 94, respectively, in the first turning after the thread T is passed through the two first thread loops L1 and the second thread loop L2 by the ligation thread hook 34.

According to the ligation device 10 of the present embodiment, the vertical walls 114 and 116 have the circumferential thread grooves 126a and 126b, respectively, at their outer peripheral surfaces. The circumferential thread grooves 126a and 126b are formed at positions corresponding to the bottoms of the radial thread grooves 118a and 118b, respectively, at the same level as the bottom of the radial thread groove 118a and 118 in the direction that the fifth rotation axis C5 extends. The circumferential thread grooves 126a and 126b are continuous with each other in the circumferential direction for positioning the thread T. The vertical walls 132 and 134 have the circumferential thread grooves 144a and 144b, respectively, at their outer peripheral surfaces. The circumferential thread grooves 144a and 144b are formed at positions corresponding to the bottoms of the radial thread grooves 136a and 136b, respectively, at the same level as the bottom of the radial thread grooves 136a and 136b in the direction that the sixth rotation axis C6 extends. The circumferential thread grooves 144a and 144b are continuous with each other in the circumferential direction for positioning the thread T. Thus, the thread T wound on the outer peripheral surfaces of the pair of vertical walls 114 and 116 and the pair of vertical walls 132 and 134 is positioned in the direction that the fifth rotation axis C5 extends and in the direction that the sixth rotation axis C6 extends, respectively, by the circumferential thread grooves 1216a, 126b and 144a, 144b.

According to the ligation device 10 of the present embodiment, of the inner walls of the radial thread grooves 118a, 118b, 136a, and 136b, the first vertical wall portions 114a, 116a, 132a, and 134a have the thread movement inclined surfaces 128a, 128b, 146a, and 146b, respectively, at their inner surfaces. The thread movement inclined surfaces 128a, 128b, 146a, and 146b extend in the first rotation direction as the thread movement inclined surfaces 128a and 128b, 146a, and 146b extend toward the bottoms of the radial thread grooves 118a, 118b, 136a, and 136b, respectively. Thus, when the first thread loop shaft 92 and the second thread loop shaft 94 are rotated in the second rotation in the loop forming rotation R2 direction, the thread T in the radial thread grooves 118a, 118b, 136a, and 136b are automatically positioned in the circumferential thread grooves 126a, 126b, 144a, and 144b.

Although embodiments of the present invention have been described above with reference to the drawings, the present invention is also applied to other embodiments.

A plastic wire material that can be tied is used as the thread T in the above-described embodiment. For example, a natural thread, a synthetic thread, a metallic thread, a composite thread or other appropriate threads is used as the thread T. The natural thread is a monofilament or multifilament thread made from plant or animal fibers. The synthetic thread is a monofilament or multifilament thread made of synthetic fibers. The metallic thread is a monofilament or multifilament thread made of metallic wires. The composite thread is made of natural fibers and synthetic fibers.

Although the ligation device 10 of the above-described embodiment has a cylindrical shape with a circular cross section, the ligation device 10 may have a prismatic shape with a polygonal cross section such as a square or hexagonal cross section.

In the ligation device 10 of the above-described embodiment, the two hooks such as the ligation thread hook and the resetting thread hook are used, but a single thread pulling member having these functions may be used.

In the ligation device 10 of the above-described embodiment, two loops L1 and L2 for ligation are formed in the thread-like member T, but three or more loops may be formed for ligation. That is, the thread loop forming portion 90 of the ligation device 10 of the above-described embodiment includes the two loop shafts, the first suture loop shaft 92 and the second suture loop shaft 94, but may include three or more suture loop shafts.

In the ligation device 10 of the above-described embodiment, as illustrated in FIG. 15, the first suture loop shaft 92 rotates in the second rotation direction R2, which is a counterclockwise direction, to form the loop L1 and the second thread loop shaft 94 rotates in the second rotation direction R2, which is a clockwise direction, to form the loop L2. Nevertheless, the first thread loop shaft 92 and the second thread loop shaft 94 may rotate in respective directions opposite to the directions in which the first thread loop shaft 92 and the second thread loop shaft 94 rotate in the above-described embodiment. In short, the first thread loop shaft 92 and the second thread loop forming shaft 94 may rotate in any directions opposite to each other.

Further, in the ligation device 10 of the above-described embodiment, the oscillation portion 30 which rotates around the rotation axis C2 is provided in the main body 14, but the oscillation portion 30 may not necessarily be provided.

In the ligation device 10 of the above-described embodiment, the opening/closing operation wires 23 for opening and closing the second jaw portion 18, the oscillation operation wires 33 for oscillation the oscillation portion 30, the second cutter operation wires 78a and 78b for rotating the second cutter 82, the thread feed rod operation wire 68 for moving the thread feed rod 64 between the retracted position and the protruding position, and the thread loop shaft operation wire 104 for rotating the first thread loop shaft 62 may be performed by a direct manual operation by an operator, or may be operated remotely using actuators that are electrically driven and controlled.

Further, in the above-described embodiment, the first jaw portion 12 is provided via the oscillation portion 30 in a state of protruding from the main body 14 in the long side direction, and the second jaw portion 18 is rotatably provided on the oscillation portion 30 so as to be openable and closable with respect to the first jaw portion 12. Nevertheless, the second jaw portion 18 may be provided 30 in a state of protruding from the main body 14 in the long side direction, and the first jaw portion 12 may be rotatably provided on the oscillation portion 30. Alternatively, both of the first jaw portion 12 and the second jaw portion 18 may be rotatably provided on the oscillation portion 30.

In addition, although not illustrated one by one, the present invention can be carried out in a mode in which various modifications and improvements are added based on the knowledge of those skilled in the art.

### Reference Signs List

10: ligation device
12: first jaw portion
14: main body
18: second jaw portion
34: ligation thread hook (thread pulling member)
38: first thread-hook passage hole (hook passage hole)
42: thread feed rod passage hole
44: notch (thread hook portion)
56: notch (thread hook portion)
54: resetting thread hook (pulling member, second thread pulling member)
64: thread feed rod
74: thread engagement portion

## Claims

1. A ligation device comprising:
a columnar main body;
a first jaw portion connected to one end of the main body in a longer side direction;
a second jaw portion connected to the one end of the main body in the longer side direction such that the second jaw portion is movable between a holding position where the first jaw portion and the second jaw portion hold a ligation object therebetween and a separated position where the second jaw portion is separated from the first jaw portion;
a thread feed rod including a thread engagement portion at a distal end of the thread feed rod, the thread engagement portion being configured to engage with a thread to be used for ligation of the ligation object, the thread feed rod being accommodated in the first jaw portion, the thread feed rod being movable between a retracted position where the thread engagement portion is located inside the first jaw portion and a protruding position where the thread engagement portion protrudes from the first jaw position when the second jaw portion is located at the holding position; and
a thread pulling member disposed inside the main body, the thread pulling member including a thread hook portion at a distal end of the thread pulling member, the thread hook portion being configured to catch the thread engaged with the thread engagement portion,
wherein the second jaw portion has:
a hook passage hole extending along the long side direction, the hook passage hole through which the thread hook portion is insertable; and
a thread feed rod passage hole through which the thread feed rod is insertable along a first short side direction, the first short side direction being a direction from the first jaw portion toward the second jaw portion and intersecting the long side direction,
wherein the hook passage hole and the thread feed rod passage hole partially intersect each other, and
wherein when the thread hook portion is located closer to a distal end of the second jaw portion in the long side direction than an intersecting position of the hook passage hole and the thread feed rod passage hole and the thread feed rod is located at the protruding position, the thread engagement portion of the thread feed rod is located downstream of the hook passage hole in the first short side direction, and is disposed at a position where the thread engagement portion overlaps the hook passage hole in a second short side direction intersecting the long side direction and the first short side direction.

2. The ligation device according to claim 1,
wherein the thread feed rod has a recessed groove at a position upstream of the thread engagement portion in the first short side direction and overlapping the thread engagement portion in the second lateral direction, and
wherein when the thread feed rod is at the protruding position, the recessed groove is located at a position where the recessed groove overlaps the hook passage hole in the first short side direction.

3. The ligation device according to claim 1,
wherein the thread pulling member includes:
a tubular body having a notch constituting the thread hook portion; and
a thread hook holding member slidable into the tubular body and catches and holds the thread between the thread hook holding member and the notch.

4. The ligation device according to claim 3,
wherein the thread hook holding member has an inclined surface at a distal end of the thread hook holding member, the inclined surface being inclined with respect to the long side direction and the second short side direction,
wherein the tubular body has an opposing surface opposing to the inclined surface, and a hooking projection linearly projecting from an edge of the opposing surface in the long side direction and in a direction away from a distal end of the tubular body in the long side direction, and
wherein the notch of the tubular body is defined between the opposing surface and the hooking projection.

5. The ligation device according to claim 3,
wherein the thread pulling member has an outer tubular member into which the tubular body is slidably inserted and into which the tubular body is projectably inserted, and
wherein the outer tubular member has an inclined end surface at distal end of the outer tubular member.

6. The ligation device according to claim 1,
wherein the thread engagement portion of the thread feed rod is a groove extending along the long side direction.

7. The ligation device according to claim 1,
wherein a first cutter configured to cut the thread is fixed to the second jaw portion, and
wherein the first cutter is disposed between the thread feed rod passage hole and the main body in the long side direction.

8. The ligation device according to claim 7,
wherein the first cutter is fixed to the second jaw portion, and
wherein the thread feed rod is movable from a first protruding position where the thread feed rod protrudes from the thread feed rod passage hole to cause the thread pulling member to catch and hold the thread to a second protruding position at which the thread engagement portion of the thread feed rod protrudes further from the first protruding position in the first short side direction when the second jaw portion is at the holding position.

9. The ligation device according to claim 1,
wherein the first jaw portion has a thread storage portion in which the thread is stored.

10. The ligation device according to any one of claims 1 to 9, further comprising a thread loop forming portion configured to form two thread loops in the thread pulled into the main body by the thread pulling member,
wherein the thread loop forming portion includes a pair of thread loop shafts, the thread loop shafts are configured to rotate in opposite directions about respective rotation axes parallel to each other and intersecting a moving path of the thread pulling member, and
wherein each of the pair of thread loop shafts includes:
a main body portion disposed apart from the moving path and configured to rotate about the corresponding rotation axis;
a pair of vertical walls protruding from the main body portion in a direction in which the rotation axis extends; and
a rod guide groove defined between the vertical walls and configured to guide a movement of the thread pulling member in the long side direction.

11. The ligation device according to claim 10,
wherein each of the vertical walls has a thread groove at a top portion thereof, and the thread groove is configured to engage with the thread pulled by the thread pulling member.

12. The ligation device according to claim 11,
wherein each of the pair of vertical walls has a pair of vertical wall portions divided in a circumferential direction by the thread groove, and
wherein a first vertical wall portion as one of the pair of vertical wall portions has:
a first thread guide inclined surface that causes the thread to move into the thread groove with rotation of the thread loop shaft in a first rotation direction; and
a thread engagement corner portion that catches the thread in the thread groove to form a thread loop around the corresponding rotation axis with rotation in a second rotation direction opposite to the first rotation direction.

13. The ligation device according to claim 11,
wherein each of the pair of vertical walls has, on an outer peripheral surface thereof, a circumferential thread groove that is at the same height as a bottom of the thread groove in the direction in which the rotation axis extends, and the circumferential thread groove continuously extends in a circumferential direction.

14. The ligation device according to any one of claims 1 to 9, further comprising a second thread pulling member configured to move between a first position where the second thread pulling member extends parallel to the thread pulling member and a second position where the second thread pulling member is coaxial with the thread pulling member.

15. The ligation device according to any one of claims 1 to 9, further comprising a coupling member that couples the first jaw portion and the second jaw portion to the main body,
wherein the second jaw portion is coupled to the main body at a proximal end of the second jaw portion via a first pin so as to be openable and closable with respect to the first jaw portion, and the first pin extends parallel to the second short side direction, and
wherein the coupling member is rotatably connected to an end portion of the tubular main body via a second pin extending parallel to the first short side direction.
